(19) Europäisches Patentamt
European Patent Office
Office européen des brevets

(11) **EP 3 957 085 B1**

(12) **EUROPEAN PATENT SPECIFICATION**

(45) Date of publication and mention
of the grant of the patent:
**17.09.2025 Bulletin 2025/38**

(21) Application number: **20719417.6**

(22) Date of filing: **15.04.2020**

(51) International Patent Classification (IPC):
*H04R 25/00* (2006.01)   *H04S 7/00* (2006.01)
*A61B 5/00* (2006.01)   *A61B 5/11* (2006.01)
*A61B 5/12* (2006.01)

(52) Cooperative Patent Classification (CPC):
**H04S 7/304; H04R 25/70;** A61B 5/1114; A61B 5/12;
A61B 5/6803; H04R 2225/81; H04R 2430/03;
H04S 2400/11; H04S 2420/07

(86) International application number:
**PCT/EP2020/060561**

(87) International publication number:
**WO 2020/212404 (22.10.2020 Gazette 2020/43)**

(54) **HEARING TEST SYSTEM**

HÖRTESTSYSTEM

SYSTÈME DE TEST AUDITIF

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.04.2019 GB 201905530**

(43) Date of publication of application:
**23.02.2022 Bulletin 2022/08**

(60) Divisional application:
**25194030.0**

(73) Proprietor: **Hearing Diagnostics Limited
Edinburgh EH42HS (GB)**

(72) Inventors:
• HORNE, Colin
Edinburgh EH42HS (GB)
• FREIGANG, Claudia
Edinburgh EH42HS (GB)

(74) Representative: **Akers, Noel James**
N.J. Akers & Co
63 Lemon Street
Truro, Cornwall TR1 2PN (GB)

(56) References cited:
US-A- 5 825 894      US-A1- 2011 019 846
US-A1- 2016 373 869

## Description

## Introduction

The present invention relates to a hearing test system and method of performing a hearing test.

## Background

Known hearing tests ascertain whether a patient is able to hear the quietest sound that a normal hearing individual ought to be able to hear. Known hearing tests subject a patient to a fixed battery of stimulus sounds (the test battery) which has sounds spanning across the frequency range, typically the frequency range of human speech (125 Hz to 8kHz).

Known hearing tests include a hearing test in which a sequence of test sounds is played to a test subject and the test subject is instructed to raise their hand when they hear the test sound. Such hearing tests may suffer from a number of problems. For example, a patient may be prone to imagining hearing sounds, in particular, in the high-stress environment of a hearing assessment. This may be particularly true for hearing impaired patients, who will often experience tinnitus (a constant ringing in the ears) which has a tendency of becoming present in a quiet environment and may become worse when focussed on.

US 2011/019846 describes hearing aids configured for directional acoustic fitting. US 5,825,894 describes a hearing evaluation and hearing aid fitting system. US 2016/373869 describes a hearing aid system.

## Summary

According to a first aspect of the invention, there is provided a device in accordance with claim 1.

The simulated source location may be characterised by one or more of a spatial locality, a laterality and/or a direction. The obtained response data may be representative of a perceived direction and/or perceived laterality and/or a perceived spatial locality of the audio stimulus.

Processing the obtained directional response data may comprise determining at least one of: a presence and/or an absence and/or an amount and/or a direction of a movement of the test subject towards and/or away from the simulated source location.

The obtained directional response data may be processed to determine a response direction. The response direction may comprise a head direction of the test subject. The response direction may comprise a direction towards which the test subject is facing.

The simulated source location may be selected such that the provided audio stimulus is provided at a stimulus direction.

The processor may be further configured to provide a plurality of audio stimuli to the test subject and process the directional response data obtained in response to providing the plurality of audio stimuli thereby to determine at least one aspect of the hearing ability of the test subject.

The processor may be configured to determine at least one statistical measure representative of the likelihood that the test subject has heard or not heard the audio stimulus based at least on said processing of said obtained directional response data.

The processor may be configured to select one or more simulated source locations for one or more audio stimuli thereby to encode information in the obtained directional response data, wherein processing said directional response data comprises extracting said encoded information.

The processor may be configured to collect said directional response data during and/or in response to producing the audio stimulus.

The processor may be further configured to receive the directional response data in real time and continually apply the source location transformation in response to receiving the directional response data in real time.

The processor may be configured to receive the directional response data at a response data rate. The processor may be further configured to continually apply the source location transformation process at a source location transformation rate based on the received directional response data. The response data rate may be substantially equal to the source location transformation rate.

The source location transformation process may be applied in response to the received directional response data being representative of at least one of: a movement of the test subject and/or a change in a response direction of the test subject.

The source location transformation process may comprise providing the audio stimulus and/or a further audio stimulus at a shifted and/or rotated source location.

The audio stimulus may be provided at a selected stimulus direction and the processor is configured to receive directional response data and process the received directional response data to determine a response direction, and wherein the source location transformation is based on an angular distance between the determined response direction and the selected stimulus direction.

The source location transformation may be such that the audio stimulus and/or the further audio stimulus is provided at a transformed angular distance from the response direction such that the transformed angular distance is greater than the angular distance between the determined response direction and the selected stimulus direction.

The source location transformation process may be characterised by one or more transformation parameters. Said one or more transformation parameters may be selected such that providing the audio stimulus and/or the further audio stimulus at the transformed simulated source location increases, decreases, maintains and/or induces a response from the test subject. The one or more transformation parameters may be varied and/or determined during the hearing test based at least on the processing of the directional response data. The one or more transformation parameters may be varied and/or selected to vary the sensitivity of the source location transformation to the response of the test subject.

The source transformation process may be applied in real-time so that the obtained directional response data is representative of an oscillating response from the test subject.

The source transformation process may be applied in real-time such that if the test subject has heard the audio stimulus, oscillations will be present in the obtained directional response data.

The source transformation process may be such that the processing resource is configured to process audio signals to provide one or more audio stimuli having corresponding simulated source locations that oscillate about a fixed or a moving spatial position in the region about the test subject. The audio stimuli may converge towards or at the fixed or moving spatial position.

The processing resource may be further configured to modify the simulated source location of the audio stimulus and/or one or more properties of the audio stimulus based on one or more pre-determined characteristics of the hearing ability of the test subject.

The directional response data may represent movement of the test subject that is substantially planar. The directional response data may be parameterised using a single parameter. The directional response data may be representative of an angular distance between a direction faced by the test subject and a direction to the simulated source location. The directional response data may comprise time-series data. The directional response data comprises head movement data.

The processor may be further configured to process the directional response data and/or data derived from the directional response data to determine one or more mathematical and/or statistical properties of the directional response data and to perform an assessment of the likelihood of the stimulus having been heard or not heard based on the determined mathematical and/or statistical properties.

The mathematical and/or statistical properties may comprise one or more of:

    a) a stationary point in the directional response data;
    b) a shape and/or a rate of change and/or a first, second or higher order derivate of the directional response data;
    c) a first and/or higher order statistical moment in the directional response data.

The at least one response feature may comprise at least one of the following:

    a) a feature representative of movement towards and/or away from the stimulus direction;
    b) a feature representative of an absence and/or delay of movement;
    c) a feature representative of oscillation about a fixed position;
    d) a feature representative of a response in a pre-determined time window following the audio stimulus;
    e) a feature representative of initial head movements towards the one or more of the audio stimuli and/or the absence of initial head movements away from one or more of the audio stimuli;
    f) a feature representative of a time elapsed between an audio stimulus and an initial movement of the test subject;
    g) a feature representative of a response direction being within an angular range about the simulated source location;
    h) a feature representative of a reversal of a direction of movement.

The audio stimulus signals may comprise binaural audio stimulus signals such that the provided audio stimulus comprises binaural audio.

The audio stimulus signals for producing an audio stimulus may comprise two audio stimulus signals that comprise an interaural level difference and an interaural time difference between the two signals such that the audio output device produces an audio stimulus that is perceived by a test subject with normal hearing ability as if is it coming from the simulated source location.

The two audio stimulus signals may be produced such that the audio output device produces an audio stimulus to each ear.

The audio stimulus signals may be processed so that at least one of:

    a) the audio stimulus has a frequency in the range 125 Hz to 8 kHz.
    b) the audio stimulus has a volume in the range -10 dB SPL to 90 dB SPL

The audio stimulus may comprise a sound at a pre-determined frequency and said processing of said obtained directional response data may comprise determining a hearing threshold of the test subject at that pre-determined frequency.

The hearing test may comprise providing a plurality of audio stimuli at a plurality of pre-determined frequencies and processing obtained directional response data for each audio stimuli thereby to determine the hearing threshold of the test subject for each pre-determined frequency.

The device may further comprise a user interface. The processing resource may be configured to provide a plurality of queries to a test subject via the user interface and receive user response data via the user interface wherein the hearing test and/or analysis of the directional response data is based at least on the user response data.

The queries may comprise at least one of the following:

a. a query relating to presence or absence of ear wax.
b. a query relating to hearing difficulties;
c. a query relating to sudden onset of hearing loss within a time period, for example, the past 24 hours, the past week, the past month;
c. a query relating to symptoms typical of tinnitus including noise, ringing in ears;
d. a query relating to test subject age;
e. a query regarding the level of social interaction by the test subject.

According to a second aspect of the invention, there is provided a system in accordance with claim 11.

The system may comprise a sensor configured to sense a response of the test subject and produce directional response data sense in response to sensing the response of the test subject during and/or in response to producing said audio stimulus.

The sensor may be configured to sense a response of the test subject and produce directional response data in response to sensing motion of the test subject during and/or in response to producing said audio stimulus.

The system may further comprise a first communication interface between the audio output device and the processing resource for communicating the audio stimulus signals to the audio output device. The first communication interface may further communicate the audio stimulus signals to the audio output device for producing the audio stimulus. The first communication interface may further communicate the audio stimulus signals to the audio output device for simulating an external sound source.

The system may further comprise a sensor configured to sense a response of the test subject and produce directional response data in response to sensing the response of the test subject during and/or in response to producing said audio stimulus.

The system may further comprise a wireless communication interface between the sensor and the processing resource configured to wirelessly transmit the directional response data from the sensor to the processing resource.

The processing resource may comprise a first processing resource and a second processing resource and a wireless communication interface between the first and second processing resources, wherein the first processing resource is configured to provide audio stimulus signals to the audio output device and receive directional response data from the sensor, and the first processing resource is further configured to communicate said directional response data to the second processing resource via the wireless communication resource.

The first processing resource may be provided remotely from the second processing resource.

The first processing resource may be further configured to process said directional response data and perform a source location transformation process comprising providing the audio stimulus and/or a further audio stimulus at a transformed simulated source location based at least on the processed directional response data. The source location transformation process may comprise performing a laterality exaggeration.

The first processing resource may be provided as part of a portable device. The portable device may be a wearable device. The portable device may be one of: a pendant, a watch, a necklace, a bracelet, sunglasses or other item of clothing. The portable device may be provided as a portable computing resource that is worn about the body, for example, a smart phone provided in a wearable holder or a smart watch. The portable device may comprise securing means for securing the portable device to a body of the test subject, for example, an item of clothing, or a belt.

According to a third aspect of the invention, which may be provided independently, there is provided a method in accordance with claim 14.

According to a fourth aspect of the invention, which may be provided independently, there is provided a non-transitory computer readable medium in accordance with claim 15.

Features in one aspect may be applied as features in any other aspect, in any appropriate combination. For example, device features may be provided as method features or vice versa.

**Brief Description of the Drawings**

Embodiments will now be described by way of example only, and with reference to the accompanying drawings, of which:

Figure 1 is a schematic diagram of a hearing test system in accordance with embodiments;
Figure 2 is an illustration of the hearing test system in accordance with embodiments;
Figure 3 is a flowchart showing, in overview, a method of performing a hearing test using the hearing test system, in accordance with embodiments;
Figure 4 is a schematic diagram illustrating a test subject and an audio stimulus produced at a simulated source location in a region about the test subject;
Figure 5 is a schematic diagram illustrating a transformation applied to a stimulus source location;
Figure 6 is a plot of transformation functions for a transformation applied to a stimulus source location;
Figure 7 is a plot of simulated response data illustrating a response of a hypothetical, idealised test subject to a sequence of audio stimuli;
Figure 8 is a schematic diagram of a method of analysis, in accordance with embodiments;
Figure 9 shows data flow between different elements of the system, in accordance with embodiments,
Figure 10 shows two examples of feature detection in simulated response data, and
Figure 11 shows an illustration of a hearing test system in accordance with a further embodiment.

**Detailed Description of the Drawings**

As noted above known hearing screening tests attempt to determine the hearing ability of a test subject by determining whether or not a test subject is able to hear the quietest sound that a normal hearing individual ought to be able to hear. In known hearing screening tests, this question is answered by subjecting the test subject to a fixed battery of stimulus sounds (the test battery), comprising sounds spanning across the frequency range of human speech (125 Hz up to 8 kHz). At the quietest levels, all clinically "normal hearing" test subjects should be able to hear and detect the sounds.

In the following, a test subject may also be referred to as a patient.

In accordance with embodiments, there is provided a system in which a test sound (an audio stimulus) is delivered to a test subject that has a spatial directionality. The delivered audio stimulus has a simulated or apparent source location in the region about the test subject. The tester instructs the test subject to turn their head to look or point with their nose towards the perceived direction of the sound, having delivered the sound to the patient, for example, via headphones. In some embodiments, the system processes directional response data (i.e. from head movements) from the test subject in response to the sounds on a trial-by-trial basis in order to verify whether, in a given trial, the test subject heard the sound. In other embodiments, the system processes response data that has been obtained over a series of trials to determine one or more aspects of the hearing ability of the test subject.

It has been found that the hearing ability of the test subject may be encoded in the coupling of the provided audio stimulus and the directional response data such that processing of the obtained response data allows for a determination of the hearing ability of the test subject. Parameters of the hearing test may be elected to maximise a) the amount of information encoded (for example, by appropriate stimulus selection and sound delivery and using, laterality exaggeration as described in the following) and b) the efficiency of the information decoding (for example, by deciding whether individual stimuli were heard, or detecting features/patterns/heuristics).

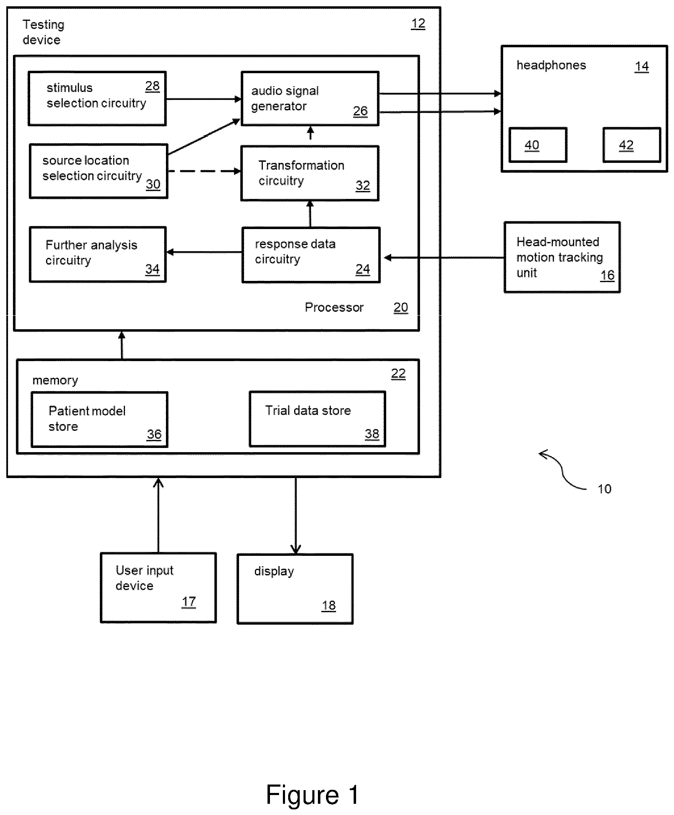

Figure 1 is a schematic diagram of a hearing test system 10 provided in accordance with embodiments. The system 10 has a hearing test device 12, an audio output device, which in the present embodiment the audio output device is a pair of headphones 14, and a response sensor, which in the present embodiment is a head-mounted motion tracking unit which may be, for brevity, be referred to as a head movement tracker 16.

The hearing test system 10 also has a user input device 17 for receiving user input and a display 18 for displaying hearing test information and general patient data, for example, test data, test results and/or hearing test instructions for a user. The hearing test device 12 has a processor 20 which is also referred to as a processing resource and a memory 22, which is also referred to as a memory resource. The hearing test system 10 is configured to be used to test at least one aspect of the hearing ability of a test subject.

The headphones 14 are configured to provide audio output, in particular, one or more audio stimuli to the test subject as part of a hearing test. The head movement tracker 16 is configured to sense a response of the test subject to the audio stimuli. In particular, the head movement tracker 16 is configured to obtain response data representative of the response of the test subject to the audio stimuli. In the present embodiment, the response data is head movement data obtained by the head-mounted motion tracking unit 16. In the present embodiment, the response data is directional response data that is representative of a head direction of response of the test subject to the audio stimulus.

The directional response data is processed by the processor 20 to determine at least one aspect relating to the hearing ability of the test subject. The directional response data may be indicative of how well the test subject is able to hear the audio stimuli. The processor 20 is configured to determine that the obtained directional response data is representative of or at least indicative that the test subject has heard or has not heard the audio stimulus.

In the present embodiment, the directional response data includes head direction data, where the head direction is measured as an orientation of the test subject's head with respect to the forward orientation. In the present embodiment, the sensed data sensed by the head-mounted motion tracking unit 16 is represented by rotation matrices that encapsulate orientation around three axes. The sensed data is pre-processed by the processor 20, for example, by the response data circuitry 24, with respect to rotations about the vertical axis to produce the response data which is represented as a time-series of a one dimensional angular representation.

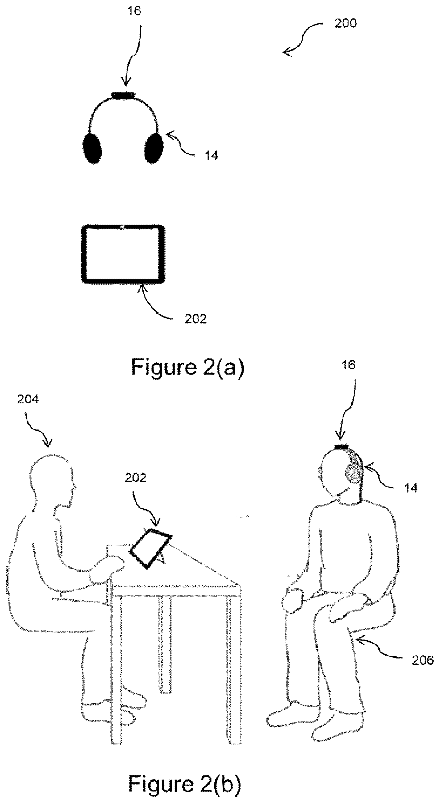

Although shown in Figure 1 as a system of separate components, some components of the hearing test system 10 may be provided together. As a non-limiting example, Figures 2(a) and 2(b) shows an embodiment of a hearing test system 200, in which the components of the hearing test device 12 of Figure 1, the user input device 17 and display 18 are provided together as a hearing test computer device 202. The hearing test computer device 202 may be, for example, a tablet computer. In addition, headphones 14 are shown together with head-mounted motion tracking unit 16. As shown in Figure 2, the head-mounted motion tracking unit 16 is attached to the pair of headphones 14. It will be understood that, in other embodiments, the motion tracker 16 and the headphones 14 are provided as a single device.

Figure 2(b) shows the hearing test system 200 in use. Figure 2(b) shows a tester 204 operating the hearing test computing device 202, for example, via the user input device. Figure 2(b) also shows the test subject (indicated by reference 206) wearing the headphones 14 and head-mounted motion tracking unit 16.

Returning to Figure 1, processor 20 has response data processing circuitry 24, which in some embodiments may also referred to as a response data processor, and audio signal generator circuitry 26, which in some embodiments may also be referred to as an audio signal generator, and response data processing circuitry 34. Although in the present embodiment, the audio signal generator circuitry 26 is shown as part of the processor 20, it will be understood that it may be provided separately.

As described in further detail in the following, the processor 20 also has stimulus selection circuitry 28, source location selection circuitry 30, transformation circuitry 32 and further analysis circuitry 34.

The memory 22 has a patient model store 36 for storing patient model data and a trial data store 38 for storing trial data. Trial data refers to audio stimulus parameters, for example, position, frequency, level used in the hearing test and the directional response data that is obtained during the trials of the hearing test.

In the present embodiment, the stimulus selection circuitry 28 is configured to select stimulus parameters. The stimulus parameters that are to be selected include frequency of the audio stimulus and volume or level of the audio stimulus.

In the present embodiment, the frequency parameters are selected to produce audio stimuli having frequencies ranging between 125 Hz to 8 kHz, thereby covering the frequency range of speech.

As described in further detail in the following, stimulus parameters for the next stimulus delivered as part of the test may be selected in accordance with a number of different methods. As a non-limiting example, these methods include adaptive sampling methods, for example staircase procedures (for example, the two-down, one-up method). More complex method may be used that seek to maximise the expected information gain (for example, as quantified by Fisher Information) of each trial.

In the present embodiment, the testing system 10 includes the presentation of test sounds, referred to as audio stimuli, over headphones 14. As described above, test subjects are asked to localise the test sounds by looking or pointing their nose towards their perceived direction of the sound source. The simulated source location selection circuitry is configured to select a simulated source location for the audio stimulus.

In the present embodiment, the simulated source locations are selected in accordance with a random pattern such that the test stimuli are presented from randomised locations in front of the patient. These sounds are delivered so as to convey interaural level difference (ILD) and interaural time difference (ITD) cues to the patient. In the present embodiment, the changes between consecutive stimulus positions are made in discrete jumps.

In further embodiments, the processor selects one or more simulated source locations for one or more audio stimuli thereby to encode information in the obtained directional response data. In such embodiments, processing the obtained directional response data includes extracting said information. This may improve reliability and/or accuracy of the hearing test. The sound source location selection may be in accordance with a known spatial pattern, for example an oscillating pattern. In some embodiments, the locations are random but recorded and then the recorded pattern is searched for in the directional response data.

At any point in time during the test, a single test stimulus is being delivered to the patient. As described in further detail below, in the present embodiment, the system continuously evaluates whether the test subject's response to this test stimulus is contributing sufficient information to justify its continued delivery. If the stimulus's continued delivery is deemed not justified, then the system terminates delivery and either commences delivery of a new stimulus or concludes the test.

If a new stimulus is to be delivered, then the processor 20, in particular the source location selection circuitry 30, selects a location for the audio stimulus subject to various constraints. In the present embodiment, a location of the stimulus will never be such that it is perceived to sound appears as if it is originating from behind the patient and commences delivery after a short interval (e.g. the stimulus directions of the test stimuli are in a region substantially to the front of the test subject). In the present embodiment, the source locations are selected to be constrained to be substantially planar, that is substantially in a transverse plane relative to the test subject.

In some embodiments, the response data is represented by rotation matrices that encapsulate head orientation around three axes. In the present embodiment, the response data is processed with respect to rotations about the vertical axis which creates a time-series one-dimensional angular representation.

The transformation circuitry 32 is configured to transform the selected simulated source position of the audio stimulus. In the present embodiment, the transformation circuitry 32 is configured to exaggerate the laterality of each test stimulus when the test subject is facing a direction in a proximal region to the simulated source position (a concept that may be referred to as "laterality exaggeration"). A number of effects may be provided by transforming the simulated source location. For example, head oscillations may be induced, fine-grained localisation accuracy may be improved and/or test subject undershoot may be reduced (the test subject is said to undershoot if they turn their head only part-way towards the stimulus's simulated source location).

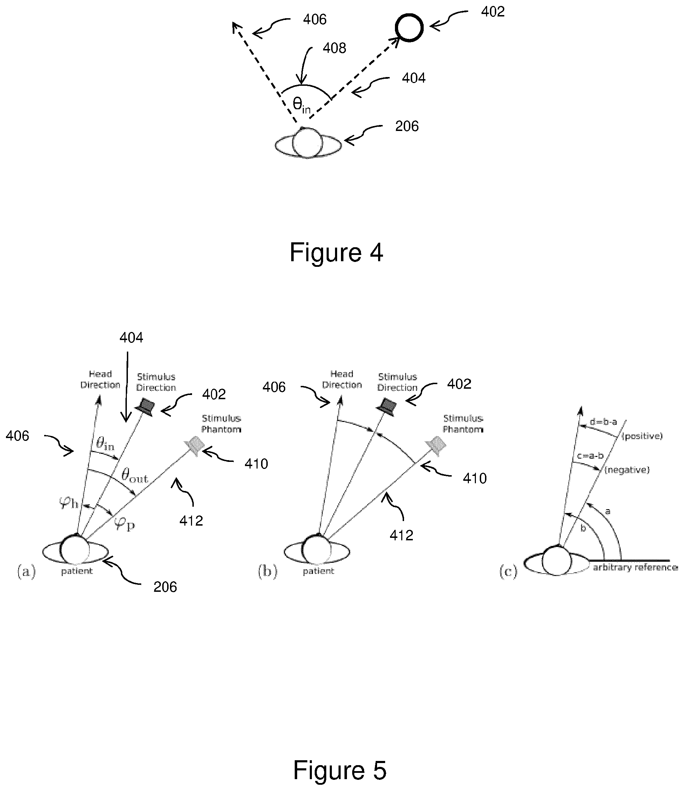

The transformation circuitry 32 is configured to receive the simulated source location parameter form the source location selection circuitry and response data from the response data circuitry. The transformation circuitry 32 is configured to perform a transformation on the source location parameter based on the received response data, in accordance with a transformation function, thereby to produce a new transformed source location. In the present embodiment, the transformation is performed in accordance with a pre-determined mathematical function. This is described in further detail with reference to Figures 5, 6 and 7.

The audio signal generator 26 is configured to provide electronic audio stimulus signals for the audio output device. In the present embodiment, the audio signal generator of hearing test device 12 provides audio signals to headphones 14 via a wired connection.

In some embodiments, the headphones 14 (or other suitable audio output device) are connected to the device 12 via a wired connection and the audio stimulus signals are electronic signals provided to the audio output device via the wired connection. In further embodiments, the hearing test device 12 is connected to the audio output device via a wireless connection and the audio stimulus signals are wireless signals sent via a wireless communication protocol. In such embodiments, the hearing test device 12 has a transmitter for transmitting said wireless signals to the audio output device which has corresponding receiving circuitry for receiving said wireless signals thereby to produce an audio stimulus. In other embodiments, the entire testing device 12 is incorporated into a headphone device. In such embodiments, user input data from user input device 17 and data to display 18 may be transmitted wirelessly.

The audio signal generator 26 is configured to receive stimulus selection parameters from the stimulus selection circuitry 28 and a source location parameter from the source location selection circuitry 30. The audio signal generator 26 is configured to produce audio stimulus signals for the headphones 14 such that the headphones 14 generate an audio stimulus having the source location.

In the present embodiment, the headphones 14 have a left headphone 40 and a right headphone 42. The audio signal generator 26 is configured to process the stimulus selection parameters and the source location parameter to produce binaural audio signals comprising a left output audio signal and right output audio signal which are then delivered to the left headphone 40 and right headphone 42, respectively. The audio output device 14 thereby produces binaural audio that comprises left output and right output.

It is known how to produce narrow band sounds having a simulated source location. In the present embodiment, the audio stimulus is generated at the simulated source location as follows. For a given a selected source location, represented by an angular distance ($\theta$) between a direction of the select source location and a direction faced by the test subject (the direction towards which the head of the test subject is facing, for example, as pointed to by the test subject's nose), an interaural level difference $f_{ILD}(\theta)$ and an interaural time difference $f_{ITD}(\theta)$ are introduced between the audio output of the left headphone 40 (left channel) and the right headphone 42 (right channel) such that the produced audio is perceived by a test subject with normal hearing ability as if it is coming from the selected source location. The level difference and time difference, $f_{ITD}$ and $f_{ILD}$ are recomputed at a high rate, so as to respond to test subject head movements.

At this point it is noted that, in order to achieve the transformation mapping, the transformation circuitry 32 is configured to transform parameter $\theta$ using a predefined transformation function $g(\theta)$ and pass parameters determined using the transformation function $g(\theta)$ so that the audio output has an applied level and time differences given by $f_{ILD}(g(\theta))$ and $f_{ITD}(g(\theta))$, respectively.

In other embodiments, the audio output device is a plurality of loudspeakers and the audio output is delivered to the plurality of loudspeakers. In such embodiments, each loudspeaker has an audio channel and the loudspeakers are provided in a ring and spatial location simulated by speaker selection and interpolation between adjacent loudspeakers.

In the present embodiment, test stimuli generated have frequencies ranging between 125 Hz to 8 kHz, thereby covering the frequency range of speech, and are delivered amplitude-modulated by a 60 Hz half-wave rectified sinusoid (100% modulation depth) in order to enhance the ITD information encoded by the envelope, so as to enable localisation of high-frequency sounds based on ITD cues. Sounds in this way processed are said to be 'transposed'. In some embodiments, each stimulus is delivered to the test subject as a rapid series of intermittent bursts so as to provide onset and offset localisation cues, which act to improve the test subject's localisation accuracy. In the present embodiment, the audio stimuli have a volume in the range -10 dB SPL to 90 dB SPL.

In the present embodiment, the head-mounted motion tracking unit 16 is a consumer MEMS inertial measurement unit (IMU), which is attached to the headphones 14 (as shown in Figure 2). Currently, an EDTracker Pro device is used, although other known devices are suitable. The IMU provides a continuous stream of directional response data reflecting the patient's head orientation, which is transmitted to the hearing test device 12 in real-time. The sensor fusion and processing of inertial sensor data into orientations is performed by a processor (on-chip) by the motion tracker 16. The response data processor 24 is configured to receive and process the directional response data obtained from the motion tracker 16. It will be understood that other devices may be used in place of the EDTracker Pro. For example, the Bosch Sensortec BNO055 may be used.

In the present embodiment, the response data processor 24 is configured to process the directional response data in real-time (during delivery of audio stimuli). The further analysis circuitry 34 is configured to perform a further analysis as part of a hearing test.

During delivery of an audio stimulus, head movement data is continuously analysed in order to test the hypothesis that the patient has heard the ongoing test stimulus. With sufficient directional response data, it may be possible to mathematically verify to within an arbitrary degree of certainty the hypothesis that the test subject did hear the stimulus. In some embodiments, a parameter representative of the required accuracy of the diagnosis (for example, no more than 1 misdiagnoses for every 1000 patients) can be provided as part of the test, in which case the test duration will be extended for as long as is necessary to confirm the hypothesis to within the required degree of certainty.

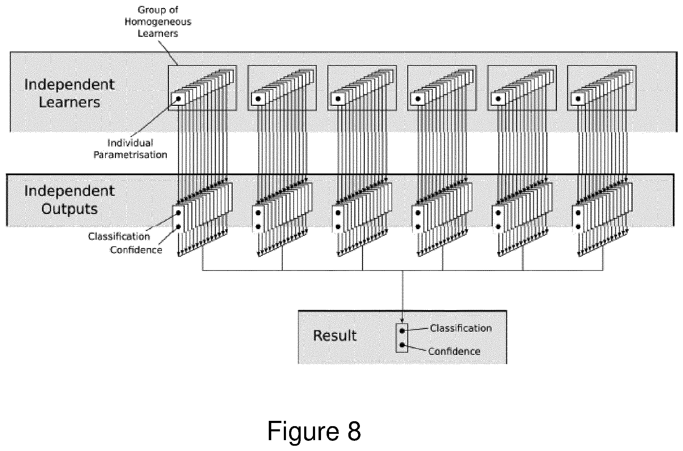

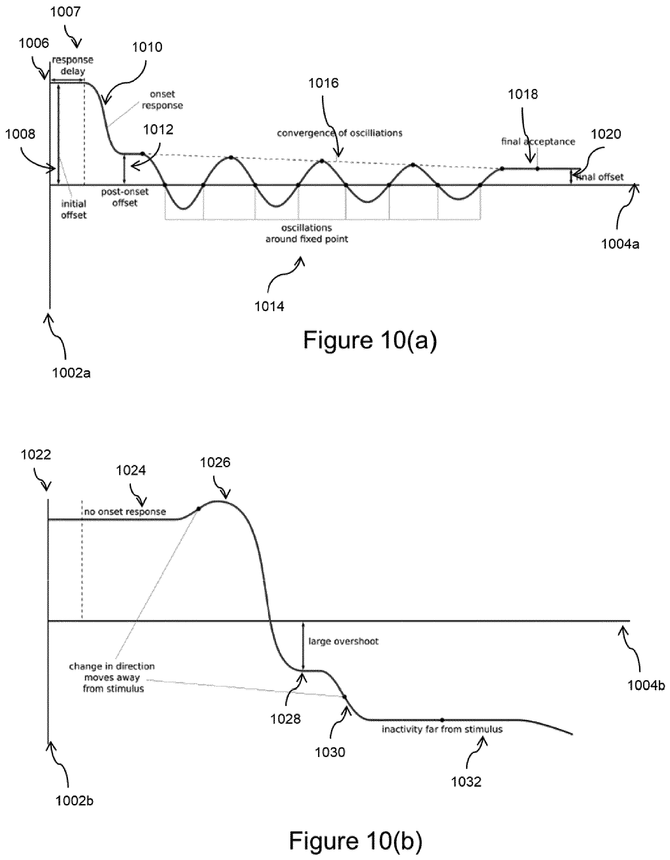

The processing of head movement data is achieved by combining the results of a collection of independent analyses, which each process time-series head movement data (the $\theta$ values) obtained from the head-mounted motion tracking unit, in order to extract discrete features. Features are notable patterns that can be recognised in the time-series data and which have associated parameters that describe specific properties of the recognised pattern (for example, time, magnitude, scale). The detection of features allows the previously continuous time-series data to be discretised into a series of events that can be analysed with traditional probability theory, radically simplifying the mathematical interpretation of the data. Analysis of directional response data and detection of features is described in further detail with reference to Figures 8 and 10.

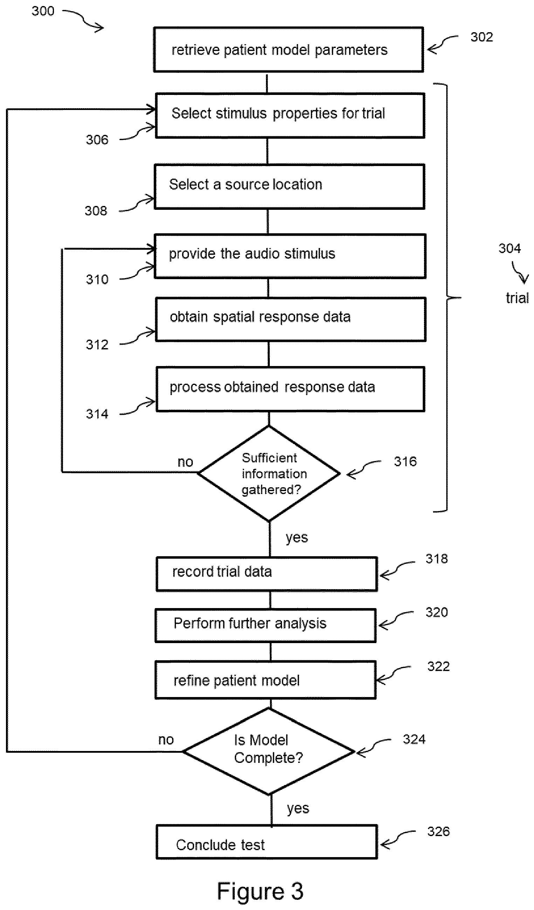

Figure 3 is a flow-chart showing a method 300 of operation of the hearing test system 10, in accordance with the present embodiment. At step 302, patient model parameters are retrieved from memory 22, in particular from patient model store 36.

The patient model represents everything that is known about the patient's hearing, for example, audiograms for both ears. The patient model may also represent characteristics of the patient's hearing that are not captured in audiograms, such as central auditory processing deficits. The model serves two functions: i) it directly informs outcomes of a hearing assessment, and ii) it informs the analysis of head movements regarding any localisation biases that should be expected, e.g., due to the patient having an asymmetric hearing loss (asymmetric hearing loss may cause the patient to be laterally biased towards their better ear).

In some embodiments, the patient model store 36 has a plurality of pre-determined patient models representative of groups of patients. For example, patient models for different patient ages or having different conditions are to be stored and loaded for the hearing test. Such pre-determined patient models may be the statistically most likely patient model for a patient of that age or group. The patient model parameters are used to determine certain parameters of the hearing test, for example, initial loudness levels and/or frequencies.

At the next step, a new trial, represented by reference numeral 304 in Figure 3, is started. A hearing test for a test subject comprises performing a plurality of trials with a test subject. During each new trial, as described below, an audio stimulus is selected and continuously delivered to the test subject via the headphone device. As the stimulus is delivered to the test subject, directional response data representative of the test subject's head movement in response to the delivered stimulus are measured. A trial ends when it is determined that enough information has been gathered or it is determined that no further information can be gathered. The steps of each trial are now described in further detail.

At step 306, properties of an audio stimulus to be produced as part of the hearing test trial are selected by the processor 20, in particular by stimulus selection circuitry 28. The properties that are selected are the frequency and level of the stimulus.

In some embodiments, the frequency and level of the stimulus are selected in accordance with a predetermined test pattern. In other embodiments, the parameters of the next stimulus can be selected using known adaptive sampling methods including simple staircase procedures (for example, 'two-down, one-up" method) to more complex methods that seek to maximise the expected information gain (for example, as quantified by Fisher Information) of each trial. In such embodiments, at least part of the trial data stored in trial data store 38 is retrieved by the processor 20 and used by the processor to select the properties of the next stimulus of the hearing test. In some embodiments, the frequency and level of the stimulus are selected based on model parameters from the patient model retrieved from the patient model store 36 in order to maximise an expected gain in information that will be realised by the patient's response.

At step 308, a simulated source location for the audio stimulus is selected by the processor 20, in particular by source location selection circuitry 30. In the following the simulated source location is also referred to as simply the source location but it will be understood that the sound is a simulated sound delivered via headphones 14.

At step 310, the parameters representative of the selected stimulus and the selected source location are provided to the audio signal generator 26 from the stimulus selection circuity and the source location selection circuitry 30.

At step 310, the audio signal generator 26 uses the selected audio stimulus parameters and the selected source location to produce audio stimulus signals for the headphones 14 such that the headphones produce audio output that has a frequency and level in accordance with the selected stimulus parameters and would be perceived by a test subject with normal hearing ability as coming from the selected source location. Step 310 may also be referred to as the sound simulation step.

At step 312, the head movement tracker 16 senses the head motion response of the test subject to the audio stimulus and produces directional response data representative of the response of the test subject.

At step 314, the processor, for example, the response data circuitry 24 processes the obtained response data. The processor processes the directional response data as it is received. The processor processes the directional response data to detect features in the directional response data and/or to perform sub analysis on the directional response data. The processor determines at least one of: a direction, a presence, an amount or an absence of movement of the test subject in response to the audio stimulus. The direction, the presence, the amount or the absence of movement of the test subject in response to the audio stimulus is used by the processor to determine that the obtained directional response data is representative of or at least indicative that the test subject has heard or has not heard the audio stimulus. Further details of the processing of the directional response data is provided below.

At step 316, the processor determines if sufficient information has been gathered. If the process determines that not enough information has been gathered, then the trial continues. In the method 300, this step is represented by moving back to step 310, where audio stimuli continues to be provided.

Steps 310 to 316 can be considered as continuous provision of an audio stimulus during which a measure of information gathered is determined. If sufficient information has been gathered by the trial, the process continues to step 318. Steps 310 to 316 may be considered as a waiting step in which the processor waits until sufficient information is gained from the current trial before ending the current trial.

At step 316, the determination of sufficient information includes determining a measure of information gained by the processing of the directional response data and comparing this measure to a threshold.

At step 318, the processor records the trial data in the trial data store 38 of memory 22. The stored trial data includes the selected stimulus parameters and source location(s) from the trial and the obtained directional response data.

At step 320, a further analysis process is performed by the further analysis circuitry 34 using the trial data stored in trial data store 38. The further analysis includes performing a mathematical optimisation to maximise consistency of the patient model across all the trials. This stage involves iteratively running the further analysis on the trial data for each trial in the trial data store. At step 322 the information from across all trials are combined to refine the patient model. The further analysis involves processing trial data of a given trial in the context of the patient model and outputting a classification and a confidence.

At step 324, the method determines if the patient model is complete or if further trials are required. If further trials are required, the method returns to step 306. If it is determined, at step 324, that the patient model is complete, the hearing test proceeds to conclude at step 326.

In some embodiments, at certain steps of the method, a timeout may occur. For example, if a timeout occurs during the trial, the method will proceed to end the current trial. If a timeout occurs at step 324, the method will proceed to step 326.

The method described above and shown in Figure 3 includes the step of generating audio stimulus signal based on selected source location. In the present embodiment, the selected source location may be transformed by transformation circuitry 32. We provide comments on this source location transformation process further below, for example, with reference to Figure 5, 6 and 7.

The method of Figure 3 is operated using system 10. The tester 204 starts the method by providing user input to user input device 17. Test information, including current status and information and results, is displayed to the tester 204 via display 18.

Figure 4 shows the test subject 206 wearing headphones 14 and head-mounted motion tracking unit 16. Figure 4 illustrates an audio stimulus produced at a simulated source location 402 in front of the test subject 206. The direction between the test subject and the simulated source location is shown and may be referred to as the stimulus direction 404.

Figure 4 also shows the direction faced by the test subject, herein referred to as the head direction 406. The obtained directional response data obtained by head-mounted motion tracking unit 16 includes data representative of the head direction 406. The obtained directional response data is representative of the time-series angular distance 408 between the head direction 406 and the stimulus direction 404. The angular distance may be represented as $\theta$ or $\theta_{in}$.

In the present embodiment, the obtained directional response data are representative of movement data and are represented as a single degree of freedom (angular distance between stimulus direction and head direction). The movement data are confined to a transverse plane and are restricted to left or right lateral movement data.

In the present embodiment, the obtained directional response data (the time-series angular distance) are processed to determine features of the directional response data, for example, to determine movement towards and/or away from the apparent source location in response to the audio stimulus. Such movement or absence of movement or characteristic of movement may be indicative that the test subject has heard or not heard the audio stimulus.

Figure 5(a) shows the operation of the transformation circuitry 32. The transformation circuitry functions together with the audio signal generator 26 to transform a source location of an audio stimulus from the simulated source location selected by the source location selection circuitry 30 to a transformed source location. The transformation circuitry 32 operates in a closed loop at a high rate. The transformed location is passed to the audio signal generator at this same rate, so that changes are immediately realised in the physical audio signal. The transformation circuitry 32 is configured to apply a transformation on a source location, referred to in the following as an initial source location, to produce a transformed source location.

Figure 5(a) shows the test subject 206. Figure 5(a) also shows an initial source location 402 and a transformed source location 410. The audio stimulus produced at the transformed source location may be referred to as a stimulus phantom. Both the initial source location and transformed source location are represented by an angular distance, in particular, by angles $\theta_{in}$ and $\theta_{out}$. These angles are measured relative to the head direction 406. Stimulus direction 404 is used to refer to the head-invariant direction of the stimulus (i.e., as represented by $\theta_{in}$ and specified by the source selection circuitry) and transformed stimulus direction 412 is used to refer to the exaggerated direction of the stimulus (i.e., as represented by $\theta_{out}$).

In the present embodiment, the transformation is a mapping applied to angular distance between head direction 406 and stimulus direction 404, represented by $\theta_{in}$. The mapping is represented by a mathematical function (transformation function) that produces a transformed angular distance, $\theta_{out} = g(\theta_{in})$. While described as transforming the angular distance, the transformation may also be considered as transforming the stimulus direction. The transformation is performed such that the interaural level and interaural time differences that are conveyed over headphones are given by $f_{ILD}(\theta_{out})$ and $f_{ITD}(\theta_{out})$, respectively.

The effect of the transform is to exaggerate the laterality of the simulated sound source, i.e. so that while the stimulus is to the subject's right, the subject will perceive it as being further to the right than it really is, and similarly for a leftwards stimulus. As described in further detail below, in the present embodiment, the transform dynamically reacts to changes in head position. The transformation thus depends upon and/or reacts-to changes in the head direction of the test subject in real-time.

In some embodiments, the parameter $\theta_{in}$ is passed to the transformation circuitry which performs the transformation, and which then passes the transformed parameter $\theta_{out}$ to the audio signal generator to generate audio stimuli.

The transformation is applied to the initial source location 402 so that the audio signal generator generates an audio signal that has a different apparent source location at the transformed source location 410. By shifting the simulated source location, the test stimulus can prompt a response that is more likely to provide useful information as part of the hearing test. In particular, the transformation shifts the source location to induce, increase and/or decrease movement of the head of the test subject in response to the stimulus.

It has been observed that, during trials of the hearing test, test subjects may often undershoot and/or overshoot in response to the audio stimulus thereby causing the response data to provide less information about the ability of the test subject to hear the response. This problem in particular with respect to undershoot may be particularly pronounced when the source location is in a region corresponding to a narrow angular range to the front of the test subject. The transformations above described facilitate this by moving the stimulus so as to avoid this region. Overshoot refers to the occurrence of the patient turning their head towards and then beyond a laterally-presented stimulus. Undershoot refers to the occurrence of the patient turning their head only partway towards a laterally-presented stimulus. This may be common due to psychological tendency to not fully commit to a head movement.

The hearing test device 12 provides for transformation of stimulus source locations (which, as discussed above, may also be referred to as a laterality exaggeration) in order to overcome, for example, undershoot of a test subject's response. The transformation may also serve to induce head oscillations or other recognisable motion patterns, and in this way, transformations may be used to embed information in patient head movements which can be recognised and extracted by the analysis algorithms.

In the present embodiment, the transformation is continuously applied in response to receiving response data in real-time. The response data is received by the response data circuitry 24 from the head-mounted motion tracking unit and the transformation circuitry applies the transform to the source location based on the received response data.

As described above, the transformation is applied to an angular distance between head direction and stimulus direction. Therefore, as the head direction changes, the transformation parameters applied to the stimulus also change thereby changing the transformed stimulus direction. Figure 5(b) shows an example of how the transformed source location is moved in response to a change in the head direction. In particular, as the head direction 406 moves towards the stimulus direction 402, the transformation is such that the transformed stimulus position 410 is moved towards the source location 402 (and hence the transformed simulated direction 412 is moved towards the stimulus direction 404).

Changes in head direction may be determined by processing the response data. It will be understood that the change in head direction may be characterised by different quantities. In the present embodiment, the response data received is a time-series of values for a single quantity which is the angle faced by the head of the test subject relative to a fixed position. The change in head direction may therefore be characterised as a change in this angle, or a change in the first derivative of this angle (the angular velocity of the head). A change in head direction may be determined by processing two or more response data points.

In the present embodiment, the response data received is a series of values for a single quantity which is the angle faced by the head of the test subject relative to a fixed position. The fixed position is the test subject's head position with respect to its straight forward orientation. By selecting a single parameter this allows the direction of audio stimuli to be restricted to within the +/- 90 degrees window around this point in the test subject's forward position. In other embodiments, audio stimuli are delivered from a full 360 degree field.

In the present embodiment, the received response data from the head-mounted tracking unit 16 comprises a series of response data points separated by a time step, each data point corresponding to the head direction of the test subject at a moment in time. The received response data from the head-mounted motion tracking unit can be considered as a data channel with a data collection frequency.

In the present embodiment, the source location is transformed continuously at a transformation frequency that is equal to the data collection frequency such that, at each time step, a transformed source location is provided based on the response data point at that time step. In the present embodiment, the transformation is time-invariant: at any given moment in time, the stimulus location is transformed on the sole basis of the head orientation at that moment in time. However, it will be understood that other embodiments will vary or tune the parameters of the transformation over the course of a hearing test.

In the present embodiment, the response data is received at a frequency of 100Hz (corresponding to a time step of response data points being received every 10 ms). Therefore the transformation is performed every 10ms. However, it will be understood that other data frequencies can be used. It will be further understood that, in other embodiments, the transformation may be applied less frequently than the response data collection frequency. For example, the response data may be averaged over a number of data points and the transformation may be applied to an averaged data point.

With regard to Figure 5(b), the obtained directional response data point can be considered as representative of a response level of the test subject to the audio stimulus at a given moment in time. For example, a response data point corresponding to a head direction at a small angle relative to the source location corresponds to a small response level and a response data point corresponding to a head direction at a large angle relative to the source location corresponds to a large response level. The processor is therefore configured to select the amount of transformation applied to the simulated source location as part of the transformation based on the response level thereby to increase and/or decrease and/or maintain the response level of the subject. In particular, the transformation is such that the audio stimulus provided to the test subject sounds as if it is coming from a further angular distance in the case when the angular distance between the head direction of the test subject and the source direction is greater than in the case when the angular distance between the head direction of the test subject and the source direction is smaller.

Figure 5(c) provides an explanation of conventions regarding negative angles (all angles have a direction of measurement that determines their sign, with anti-clockwise angles being positive; this is consistent with all directions being measured as angles from an arbitrary fixed reference in an anti-clockwise direction, and with relative angles being given by the appropriate arithmetic).

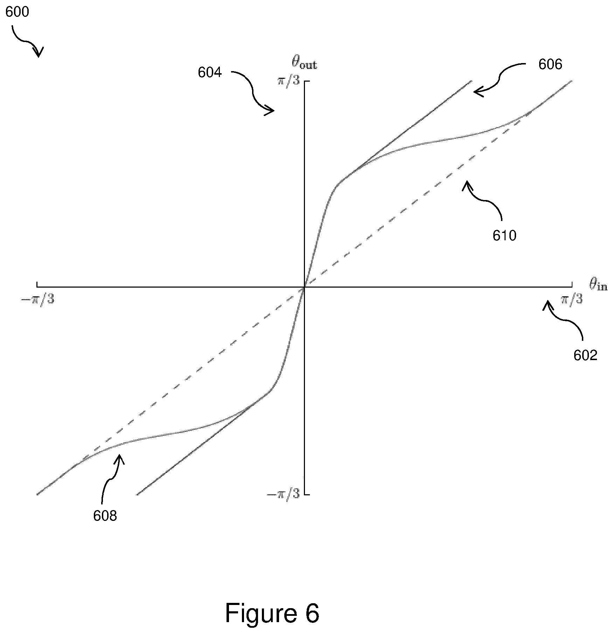

A plot 600 of non-limiting examples of transformation function $g(\theta_{in})$ is shown in Figure 6. Figure 6 shows the transformation function $\theta_{out} = g(\theta_{in})$ for two different parameterizations of g.

The x-axis 602 represents the angular distance 408 between stimulus direction 404 and head direction 406 (represented by $\theta_{in}$). The y-axis 604 represents angular direction between head direction 406 and transformed stimulus direction 412 (represented by $\theta_{out}$).

Figure 6 shows two different parameterisations (first curve 606 and second curve 608) of the transformation function $\theta_{out} = g(\theta_{in})$. The first curve 606 and second curve 608 vary in their rate of return to the identity mapping 610, represented by dashed line ($\theta_{out} = \theta_{in}$). As can be seen from Figure 6, both transformation curves are non-linear.

In the present embodiment, a suitable transformation mapping is represented as a mathematical function accepting at least three parameters to allow control of:

i) the gradient at zero,
ii) the maximal offset from the identity transform ($\theta_{out} = \theta_{in}$)
iii) rate of return to the identity transform.

Any definition for transformation function g that that encapsulates these parameters may be used.

A greater offset from the identity transform has the effect of magnifying the head oscillations induced by the transform. A steep gradient at zero ensures head oscillations are induced even when the head direction 406 is very close to the stimulus direction 404. A return to the identity transform removes laterality exaggeration for highly lateral sounds, which may be desirable if the test subject is not prone to undershoot, providing the test subject does not become confused by the resulting non-monotonicity of the apparent stimulus movement.

The parameters of the transformation function can be fixed, having had their optimal values learnt empirically during the product's testing, but it may be desirable for them to be slowly varied during the course of a patient assessment in order to adapt to the needs of the test subject. In some embodiments, the parameters of the transformation are selected based on patient model parameters. As a non-limiting example, a patient who is prone to excessively pronounced undershoot may need a greater maximal offset from the identity transform.

In some embodiments, the transformation parameters are varied and/or selected to vary the sensitivity of the transform (i.e., its gradient at zero) to the response of the test subject. The tuning of the transformation's parameters on the basis of head movements occurs over a long time-course, for example, over the course of the hearing test and/or over the course of one or more trials. In some embodiments, the transformation parameters are selected to set the sensitivity of the transform to head movements on the basis of recognizing that the transform has not previously been successful in inducing head movements. As a non-limiting example, the transformation applied to the head direction may be too close to identity for a test subject and thus the test subject is not induced into moving their head towards the stimulus direction. Therefore, a parameter of the transformation is varied so that the transformation applied provides a larger angular rotation to the stimulus direction. Similarly, sensitivity may be varied in response to repeated undershoot.

Although described as an angular transformation, in other embodiments, the transformation may be modelled for example, as a shift and/or be considered as a rotational transformation. The shift and/or rotation is relative to the present head direction of the test subject.

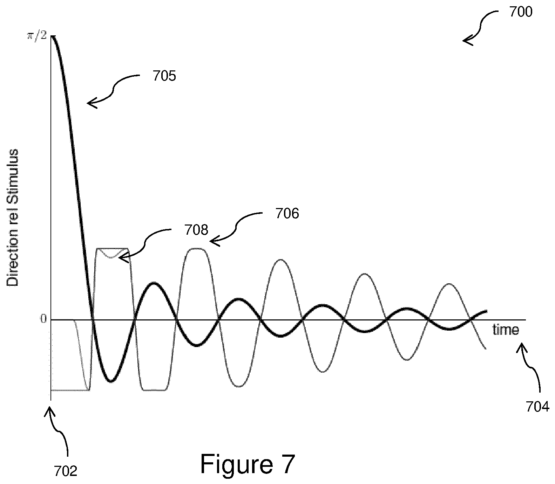

Figure 7 shows a plot 700 of simulated directional response data from an idealised patient (represented as a time series of angular distance between head direction 406 and stimulus direction 404 or transformed stimulus direction 412). The data illustrates a hypothetical, idealised test subject's response to an audio stimulus with varying transformed source locations. The source locations are transformed by the transformation circuitry 32 thereby to induce an oscillation in head movement. The y-axis 702 represents angular distance. For curves 705, described below, the y-axis 702 represents angular distance between head direction 406 and stimulus direction 404. For curves 706, 708 described below, the y-axis 702 represents angular distance between stimulus direction 404 and transformed stimulus direction 412. The x-axis 704 represents time.

Figure 7 shows three curves illustrating an idealised example of the relationship between the obtained hypothetical response data and the simulated source location. The first curve 705 shows head direction relative to the stimulus (the angular distance between head direction 406 and stimulus direction 404). The second curve 706 and third curve 708 represent the transformed source location (the angular distance between transformed stimulus direction 412 and stimulus direction 404). The second curve 706 corresponds to the transformation map 606 shown in Figure 6 and the third curve 708 corresponds to the transformation map 608 shown in Figure 6.

Figure 7 shows that the transformation applied to the source location can induce head oscillations as the test subject repeatedly overshoots the audio stimulus. It is observed that the distance between the head direction 406 and the transformed stimulus direction 412 is always greater than the distance between the head direction 406 and the stimulus direction 404, which may constantly motivate the patient to overshoot the stimulus. Head oscillations are thus induced as the test subject constantly seeks to correct the overshoot by turning in the opposite direction.

Head oscillations can be achieved if the subject repeatedly overshoots the stimulus (and possibly undershooting the transformed stimulus). If the subject undershoots the source location then head oscillations are not induced. In such circumstances, the parameters of the transformation mapping can be tuned to in an effort to induce overshoot in later presentations).

Head movement data for a single stimulus trial are processed and analysed to test the hypothesis that the test subject heard the stimulus. It will be understood that the analysis of head movement data may be performed using different methods. In particular, in some embodiments, rather than testing the hypothesis that the test subject heard the stimulus on a trial by trail basis, a machine learning approach may be used which does not explicitly encode whether each stimulus was heard, but rather operates monolithically on the entire head movement data across the entire test's duration (i.e. across the response data collected over multiple trials in response to different stimuli).

In further detail, in such embodiments, rather than deciding for each individual trial whether the stimulus was heard, a machine-learning approach is provided that does not explicitly encode whether each stimulus was heard, but rather operates monolithically on the response data collected over the hearing test (i.e. multiple trials) in order to predict the hearing ability of the test subject.

In accordance with embodiments, the laterality exaggeration mechanism (the transformation described above) moves the sound source as a direct reaction to the movements of the test subject's head. Empirically, it has been found, that this may tend to create oscillations as a result of the subject continuously attempting to correct their head orientation following the sound source's movement. However, if the subject does not make these corrections (if the subject maintains a static head orientation) then no oscillations are produced. It has been found that oscillations may be an emergent property of laterality exaggeration.

In other embodiments, head movement (for example, oscillations) are induced independently to the transformation. In such embodiments, head movements are induced that match the sound source movements (i.e., in terms of cross-correlation or otherwise).

In the present embodiment, the analysis of head motion data is achieved by combining the results of a collection of independent sub-analyses, each of which makes an individual prediction as to whether or not the stimulus was heard. The predictions are combined into a single unified prediction that is more reliable than any individual prediction in isolation.

In this way, the predictions of the sub-analyses with respect to whether a sound was heard/ not heard may be made on the basis of easily tested heuristics that would not be individually reliable, but which when combined, empirically produce strong predictive accuracy. The advantage of this approach is that heuristics may be combined without understanding of how they interact with one-another, avoiding the need for an all-encompassing mathematical model of how the patient moves their head in response to stimuli. Furthermore, it may be easy to add additional heuristics (in the form of additional sub-analyses) at a later stage.

While a combination of sub-analyses is described for the present embodiment, it will be understood that, in other embodiments, different analysis is performed to make a prediction or other statistical measurement that the test subject has heard the stimulus. As a non-limiting example, a convolutional neural network may be used to categorise time-series response data into heard/not heard categories. A classifier for classifying response data or data derived from the response data into at least heard or not heard may be trained and then used to classify further obtained response data.

This methodology is well aligned with a known framework of ensemble learning, in which each of the sub-analyses form independent learners (or weak learners). Providing each independent learner is better than random chance in predictive accuracy, the outputs across all learners can be combined to form a single ensemble prediction that can be made arbitrarily good given enough independent learners. In ensemble learning terminology, the independent learners are homogeneous if all learners apply the same logic and differ only in their parameters, or heterogeneous if they also differ in their logic. In the context of the sub-analyses, each use independent logic representing independent heuristics, and are thus heterogeneous. However, each sub-analysis is in some way parametrized (e.g., representing some threshold value that must be exceeded for a positive prediction to be made) and it may be beneficial for a single sub-analysis to be instantiated with multiple different parameter configurations, thereby giving rise to a multitude of homogeneous learners. The final result is thus a hybrid approach, combining heterogeneous groups of homogeneous learners.

Figure 8 shows a plurality of homogeneous learners that relate to a single sub-analysis which are systematically generated using any of a variety of known methods for creating diversity in parameters. A suitable method includes bagging (or bootstrap aggregating), wherein the parameters of each individual learner are optimised (via any suitable optimisation method, such as by gradient descent) by using as training data only a random subset of the samples available in a training set (which is a large collection of directional response data, in this case, time series angle data for which it is already known whether the patient could hear the stimulus). This ensures that each learner is optimised using different training data, and thereby the resulting parametrizations of each learner differ. Further discussion of individual sub-analyses is provided below with reference to Figure 10.

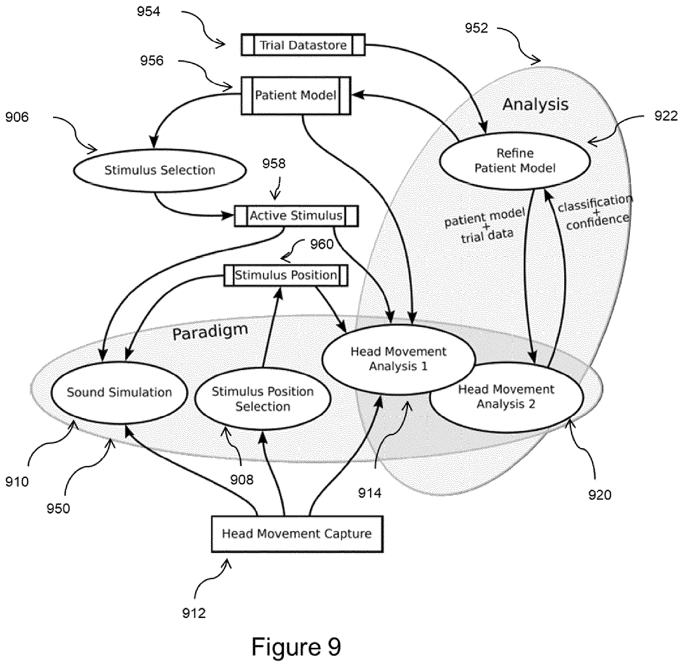

Figure 9 is a schematic diagram illustrating data flow and storage between different elements of the hearing test system 10. Figure 9 shows the flow of data between processes and data stores. Processes are shown as ellipses, external data sources are shown as rectangles, and program variables are shown as rectangles with side-bars. Arrows represent the directional flow of data. Note that the dataflow for populating the trial data store 38 with head-movement data and stimulus parameters is omitted for clarity.

Several processes are shown which substantially correspond to method steps of method 300: stimulus selection 906 (corresponding to select stimulus properties for trial step 306), stimulus position selection 908 (which substantially corresponds to selecting source location step 308), sound simulation 910 (which substantially corresponds to provide audio stimulus step 310), head movement analysis 1, represented by numeral 914 (which substantially corresponds to process directional response data step 314) and head movement analysis 2, represented by numeral 920 (which substantially corresponds to perform further analysis step 320) and refine patient model step 922 (which substantially corresponds to refine patient model step 322).

A number of the above processes are grouped into a paradigm grouping 950 and an analysis grouping 952. The paradigm group includes processes 908, 910, 914 and 920. The analysis grouping includes processes 914, 920 and 922. It is seen that processes 914 and 920 belong in an overlap group between paradigm grouping 950 and analysis grouping 952.

Figure 9 also shows data source: head movement capture 912, which substantially corresponds to data obtained during obtain directional response data step 312. Figure 9 also shows the following program variables: trial data store 954, patient model 956, active stimulus 958 and stimulus position 960.

Turning to Figure 10, Figure 10(a) and Figure 10(b) each show an illustrative example of feature detection from the processing of response data.

In the present embodiment, the data signal that is processed and obtained from the head motion tracking unit 16 is pre-processed before any further processing for detection of features is performed. The pre-processing includes processing the data to have a scale dimension $\sigma$ with $\theta_\sigma = \theta * f_\sigma$ where

$$f_\sigma(t) = \frac{1}{\sqrt{2\pi\sigma^2}} e^{-\frac{t^2}{2\sigma^2}}$$

i.e. $\theta_\sigma$ is the convolution of $\theta(t)$ with a Gaussian of standard deviation given by the scale parameter.

Figure 10(a) and Figure 10(b) show idealised head movements corresponding to the case of a test subject hearing the audio stimulus and idealised head movements corresponding to the case of a test subject not hearing the audio stimulus. Figure 10(a) and 10(b) illustrate a selection of response features that are detected by processing directional response data. In both Figure 10(a) and 10(b), the y-axes (1002a, 1002b) are representative of the angle $\theta$ which is representative of the angular distance between the head direction 406 of the head relative to the stimulus direction 404, as shown in Figure 4. The x-axes (1004a, 1004b) are representative of time.

Turning to Figure 10(a), an initial stimulus is produced (stimulus onset 1006) at an initial simulated source location as described in detail above. The test subject hears the stimulus and reacts after a short delay (response delay 1007) and then produces the first discrete head movement (referred to as the onset response 1010). The absolute deviation (measured by the magnitude of the angular distance) between the head direction 406 and the stimulus direction 404 at the time of the stimulus onset is referred to as the initial offset 1008. The first discrete head movement (onset response 1010) undershoots the stimulus, that is the test subject does not move their head sufficiently far towards the simulated source location of the stimulus.

Figure 10(a) also shows the absolute deviation between the head direction 406 and the stimulus direction 404 immediately following the onset response (the post-onset offset 1012).

A series of transformations are applied to the source location based on the transformation mapping and the raw directional response data to produce a series of stimuli at transformed stimulated source locations. As a result of the transformations, oscillating head movement is induced in the test subject (oscillations around a fixed point 1014, the fixed point being the un-transformed source location).

The oscillating head movement consists of a series of consecutive attempts by the test subject to correct the overshoot, each of which may be considered as a further overshoot, creating strong oscillations around the un-transformed stimulus position. Eventually, the test subject converges sufficiently close to the stimulus so as not to be able to detect his/her deviation from it (even in the presence of further transforms), and thus settles upon this final head position until the trial's end (referred to as final acceptance 1018). The absolute deviation from the stimulus at the time of a detected final acceptance is referred to as final offset 1020.

In the present embodiment, the un-transformed source location is never delivered to the subject as it always first undergoes transformation. In the present embodiment, the audio stimuli are not oscillating however, may be induced by the head movements of the test subject. For example, if the test subject keeps their head fixed, the stimulus will remain fixed and will not oscillate. The stimulus therefore only oscillates if the test subject response oscillates.

In the above described embodiments, the un-transformed source location is also referred to as the initial source location. However, it will be understood that in some embodiments, an audio stimulus is never delivered to the test subject from the untransformed source location, as a transformation is applied before the first audio stimulus is provided.

Figure 10(b) shows an example of idealised head movements corresponding to the case of the test subject not hearing the stimulus. The stimulus onset is at time 0 (shown at 1022). In contrast to Figure 10(a), the test subject does not hear the stimulus and therefore does not react for some time (absence of onset response 1024). When the test subject does respond, the response is not necessarily in the direction of the stimulus location. For example, the test subject first moves away from the stimulus (1026). Then, by chance, the test subject's next head movement is towards the stimulus but overshoots considerably (greater than would be accounted for, for example, by an applied transform). The overshoot is shown as a large overshoot 1028 in Figure 10(b). The next head movement 1030 is further from the stimulus. A period of inactivity 1032 then follows, far from the stimulus direction, but does not continue until the end of the trial, by chance being interrupted with the patient again turning away from the stimulus.

A description of response features is provided in the following. In accordance with embodiments, the processor is configured to perform an assessment of the likelihood of the audio stimulus having been heard by the test subject in dependence on one or more statistical properties of the directional response data and/or data derived from the directional response data.

In the present embodiment, processing of the response data includes performing one or more mathematical operations on the response data to determine one or more mathematical and/or statistical properties of the response data. In some embodiments, the mathematical operation includes determining a stationary point in the response data. In some embodiments, a rate of change and/or a first, second or higher order derivative is determined. In some embodiments, the value of a first, second or higher order derivative at a specific point, for example, at a stationary point, is determined.

In some embodiments, a shape or a change in shape of the response data is determined. Further statistical properties may be determined, for example, a first and/or higher order statistical moment in the response data may be determined. In some embodiments, a mathematical transformation is performed on the response data prior to and/or during determination of response features. The mathematical operations may include performing an average or determining a statistical measure of the response data.

The following examples are provided as illustrative and will be understood to be non-limiting.

As a first non-limiting example, first and/or higher-order statistical moments of the angular component of stationary points in the response data (that is, rotations around a dominant axis) and its first, second, and higher-order derivatives are analysed. This analysis may be performed by hypothesis testing or simple range comparisons with respect to their sampling distribution for an expected central tendency that is hypothesised to be indicative of the stimulus having been heard.

Zero-crossings in the first-order derivative of the time-series response data correspond to head reversals of the test subject. If the stimulus was heard, the angular components of head reversals will cluster around the stimulus position, and so the first order moment of zero-crossings in the first-order derivative will be in the neighbourhood of the stimulus position.

In further embodiments, a mathematical transformation is performed on the response data before or as part of determining features. For example, the response data may be transformed, scaled or rotated to a different co-ordinate space in which features may be determined. The mathematical transformation may include a convolution with a kernel. The transformation may be applied in addition to or in place of performing further mathematical features, for example, instead of taking one or more derivatives of the response data.

A further feature relates to a delay (or absence of a delay) with respect to the time at which one or more features occur following the time at which a stimulus is first presented at a new position. It has been found that in a high number of cases, when a new stimulus is presented from a new position, there may be an immediate (or rapid) initial movement towards the new stimulus position. Such a delay (or absence of a delay) may be a dominant feature in deciding whether the stimulus was heard.

In the following, a further description of some possible response features is provided:

*Discrete head movement*

A single discrete head movement (i.e. a step) with a particular scale. Any method of detection can be used that detects steps (or slopes) across multiple scales. Well known methods exist in step detection and its two-dimensional equivalent of edge detection. Note that there are several obvious discrete head movements in Figure 10(b), but in addition to those, there is another discrete head movement at a much larger scale corresponding to the patient's overall head movement throughout the duration of the trial (i.e., once the curve is sufficiently smoothed, all obvious discrete head movements blur together and combine into a single large discrete head movement).

*Inactivity*

A time-period during which no head movements have occurred at a particular scale.

*Final acceptance*

Inactivity that is detected to overrun with the end of the trial.

*Final offset*

The absolute deviation from the stimulus at the time of a detected final acceptance.

*Head oscillations*

A series of detected discrete head movements that alternate in direction and that each result in the head midline crossing over the stimulus location. The definition allows for each oscillation in the series to have different scales (i.e., periods). The oscillations occur as a result of the test patient wanting to correct the perceptual spatial discrepancy between a current head position and the transformed simulated source location, and depends on the spatial hearing ability of the test subject.

*Head oscillations converging towards a fixed point*

Same as previous, but with the additional constraint that the magnitude of the discrete head movements must be progressively decreasing.

*Onset response*

The first detected discrete head movement following stimulus onset.

*Initial offset*

The absolute deviation, or the magnitude of angular distance between, the head direction and the stimulus direction at the time of the stimulus onset.

*Post-onset offset*

The absolute deviation in the head direction and the stimulus direction immediately following the onset response.

As described with reference to Figure 8, each sub-analysis processes directional response data. In the present embodiment, these analyses process the time-series $\theta$ values to detect pre-determined features. Using these detected features, and on the basis of these features, the patient model parameters, the stimulus parameters, and some independent heuristic, a prediction as to whether or not the stimulus was heard can be made.

The patient model is taken into account when making predictions by applying a compensating transformation for any known lateralization bias the test subject may exhibit. In the present embodiment, the reference value representative of a test subject facing towards the stimulus is rotated from 0 to r, where r is an angular rotation of the head that represents the extent of lateralization bias affecting the patient. The patient is thus considered to be looking at the stimulus if $\theta = r$. The parameter *r* may be stored as a patient model parameter.

The decision logic of a selection of sub-analyses are described below (i.e., each description corresponds to a single sub-analysis). For clarity, the compensation of lateralization bias is omitted, but may be implemented as described above. Each sub-analysis is represented by a number of feature parameters.

*Response Delay Analysis*

Predicts stimulus was heard if the earliest-detected discrete head movement following a stimulus onset occurs within an expected time window and is in the direction of the stimulus. Parameterised by the duration of the time window, the minimum scale of the head movement, and the feature tolerance (the amount of tolerance to deviations from an ideal representation of the feature).

*Improvement in offset Analysis*

Predicts stimulus was heard if the final offset improves upon the initial offset. Parameterised by the threshold by which the offset need be improved.

*Oscillation detection Analysis*

Predicts stimulus was heard if head oscillations are detected (as illustrated with reference to Figure 7). Parameterised by scale, feature tolerance, and the minimum number of consecutive oscillations necessary for detection.

*Convergence of oscillations Analysis*

Predicts stimulus was heard if oscillations converging towards a fixed point (see features) were detected. Parameterised as in oscillation detection (as described above previously), in addition to feature tolerance and how much magnitude decay is necessary.

*Final offset Analysis*

Predicts stimulus was heard if a final offset (see features) is detected and the offset is better than some threshold amount. Parameterised by minimum duration of the inactivity, the threshold amount, and feature tolerance.

*Post-onset offset Analysis*

Predicts stimulus was heard if a post-onset offset (see features below) is detected and improves upon initial offset. Parametrised by feature tolerance and magnitude of required improvement.

*Consistency of head movements Analysis*

Detects all discrete head movements (see features) and attributes each as being either 'correct" or 'incorrect" on the basis of whether the movement began by turning towards or turning away from the stimulus (irrespective of whether overshoot occurred). Predicts stimulus was heard if the ratio $n_{correct}/n_{incorrect}$ exceeds a threshold value (where $n_{correct}$ represents the number of correct head movements; similarly, for $n_{incorrect}$).

Parameterised on the threshold ratio and feature tolerance. Consistency refers to changes in head movement direction that always result in the head initially moving towards the stimulus. Alternatively, this may mean that the subject never accelerates away from the stimulus.

*Quasi-variance after n-th discrete head movement Analysis*

Predicts stimulus was heard if the integrated squared head deviation from the stimulus between the end of the n-th discrete head movement and the end of the trial is less than some threshold value. Parameterised on the threshold value and n.

That is,

$$\int_{t_0}^{T} (\theta(t) - r)^2 \, dt < \phi^2$$

where t is the time of the end of the n-th discrete head movement, T is the time of the end of the trial, $\varphi$ is the threshold, and r is the stimulus position after accounting for lateralisation bias (i.e., 0 if no bias present).

Note that t is invariant to r and thus the effect of changes to r can be computed without needing to re-compute the integral. Changes to the patient model can be handled computationally efficiently without needing to reprocess the entire time-course of θ.

*Inactivity far from stimulus*

Predicts stimulus was ***not*** heard if inactivity was detected further than a threshold distance from the stimulus. Parametrised on the threshold distance, the threshold duration of inactivity, and feature tolerance.

*No onset response Analysis*

Predicts stimulus was ***not*** heard if no discrete head movements are detected during a time window following stimulus onset. Parameterised by the minimum scale of the head movement and the duration of the time window.

As described above, analysis or sub-analysis of the directional response data may include detection of discrete features in the directional response data in the time series data signal. Some features depend upon the concept of scale, which represent the amount of temporal blurring applied to the data signal prior to the feature being detected.

The patient model is continuously updated and refined as more head movement data becomes available, with the goal of finding a model that maximises consistency between the models, and the predictions made from analysing head movements across all stimulus trials using the model. In this way, the patient model and the predictions are mutually dependent: the patient model directly affects the predictions of the analysis of head movements, and the predictions directly affect which model maximises consistency with those predictions. This mutual dependency converges upon a steady state solution when further refinement has no effect on the predictions of the analysis due to optimal parameters having already been found. At this point, the model is hypothesised to best represent the test subject's true hearing abilities, to the extent that is permitted by the model and the data available. Any suitable optimisation method can be used for finding this solution (e.g., simulated annealing).

The model and the analysis are consistent for a given trial if the predictions made by the analysis are consistent with the audiograms encoded by the model (i.e., the analysis predicting the test subject heard a sound is consistent with the model if and only if the audiograms indicate that the sound is above the patient's auditory threshold at the sound's frequency).

At any point in time, a confidence value can be computed representing the stability of the patient model with respect to the input data (that is, a representation of how much the data from a single stimulus trial affects the outcome of refining the model). High levels of stability indicate high confidence: if the model truly represents the test subject's hearing abilities, then its fitting should not pivot upon any single stimulus trial.

The hearing assessment continues until some threshold level of confidence is achieved, or until a timeout is reached (in which case the results of the hearing assessment will be inconclusive).

It will be understood that the hearing test system 10 may be used for a number of different applications. The system of the present disclosure relates to hearing testing products that may be used by a number of different users. In some embodiments, the system is intended to be used by a tester who is a primary health provider or social care worker untrained in audiology (for example, a GP, practice nurse or pharmacists) in order to determine whether the test subject has hearing problems that require further assessment by a trained audiologist.

*Screening*

The first application considered is screening for the presence of hearing loss. The system may offer the following advantages over known hearing test methods: I) of greater reliability, ii) objectively tell whether sound was heard on the basis of whether head movements relate to true stimulus position, iii) can estimate probabilities or measures of confidence regarding reliability of test result, iv) robustness against background noise, v) spurious head movements arising from the test subject responding to background noises (for example, a door closing) do not product false results by virtue of the quantitative analysis of head movements. Furthermore, spatially localised sounds (i.e., such as are being used as the stimulus in the present paradigm) may be easier to differentiate from omnipresent background noise than omnipresent sounds (i.e., such as used by the current standard paradigms).

Further advantages are that the system may not require a soundproof booth. For example, in low-level noise environments (for example in a private room in an office environment), robustness against background noise means no soundproof booth will be required. The system also provides robustness against tinnitus, in that tinnitus is common in hearing-impaired individuals and creates significant problems in screening due to patients responding to their tinnitus rather than to the test stimuli. The present system and method may be robust to tinnitus due to being able to objectively infer whether a sound was really heard.

*Clinical Audiology (Diagnostics)*

A further application of the described method and system is in the field of clinical audiology. In this field, the system can be used in a diagnostic context for the assessment of audiograms or evaluation of central auditory processing deficits. Benefits may be the same as for screening. However, it is likely that in clinical audiology, a soundproof booth would be required as diagnostic applications assess hearing sensitivity at lower sound intensities than those used in screening, which may not be audible if background noise is present.

*Paediatric Audiology*

The system may also be applied in the field of paediatric audiology. In this field, it can be prohibitively difficult to obtain audiograms with known methods due to poor attention span and self-discipline in young children. The benefits to paediatric audiology may be as described above, in adults. In addition, the on-going spatially-localisable stimulus and the transformation may create an engagement element that may keep the child focused.

Additionally, further features may be added to the hearing test method specific for paediatric audiology. As non-limiting examples, if at the end of each trial the stimulus transiently increases in level before morphing into either a fanfare (or other rewarding sound) if the stimulus was well-localised, or a dissatisfying 'squelch' if not, then the paradigm has a feedback and game element. The child can therefore assess their own performance and gains auditory rewards for good performance, increasing engagement.

Hearing Aid Fitting

The system may also be applied in the field of hearing aid fitting. In this field, it is known that hearing thresholds derived from the conventional pure-tone audiogram do not reliably predict hearing aid settings of a patient. As a consequence, audiologists are forced to figure out the best hearing aid fitting by applying time-consuming and inefficient trial and error procedures. The present system provides a more holistic hearing testing approach that may be beneficial for deriving parameters for hearing aid fitting that allow an automated strategy for preparing hearing aid fittings for patients. The system may be applied for fitting conventional hearing aids as well as emerging over-the-counter hearing aids.

A skilled person will appreciate that variations of the enclosed arrangement are possible without departing from the invention.

For example, the system, as shown in Figure 2, is packaged as pre-installed software on a tablet computer. However, it will be understood that the processor could form part of any suitable computing device (desktop, tablet, laptop or similar). It will be understood that the processor could form part of a mobile computing device, for example, a tablet or a smartphone. It will be further understood that the processor could form part of a single board computer, such as, for example, the Raspberry Pi computer.

In the above described embodiment, the spatial response sensor is a head-mounted motion tracking unit. It will be understood that, in other embodiments, the response sensor can be any suitable response measuring device. For example, the response sensor could be an eye tracking device configured to obtain eye direction data and/or eye direction data. The response sensor could be a user operated device configured to receive user input representative of a response. The response sensor could be one or more motion and/or position sensors wherein the sensor output is processed to provide said directional response data. The response sensor could be a hand held or a body-mounted controller that a test subject uses to point or gesture to the perceived source location. Further devices that may be used include a flat sliding-scale (for example, a clock-like device that lies flat on a table where a single arrow can be moved to point to the suspected sound source location).

While in the above described embodiments, head-mounted motion tracker is described to obtain head movement data, other directional response data sensors can be used. For example, other devices for obtaining head movement data, or eye-trackers for obtaining eye movement data, or other devices for obtaining hand and/or body movement data can be used. In other embodiments, the response data may be spatial response data that represents position a body and/or part of the body and/or object in space.

In other embodiments, the headphones 14 may be any suitable audio output device headphone device, for example, head phones, ear phones, a virtual reality headset or loudspeakers.

As a further example, in the above described embodiments, audio stimuli provided at a fixed position and the sound delivery mechanism (including the transformation) give the impression of moving audio stimuli.

In other embodiments, a plurality of audio stimuli having corresponding simulated source locations selected in accordance with a pre-determined source location pattern are provided. The source location pattern is used when processing the obtained directional response data. The source location pattern is searched for in the obtained directional response data. In further embodiments, information may be embedded in the simulated source locations retrieved during processing of directional response data. In such embodiments, the motion pattern is encoded within the stereo stimulus waveform.

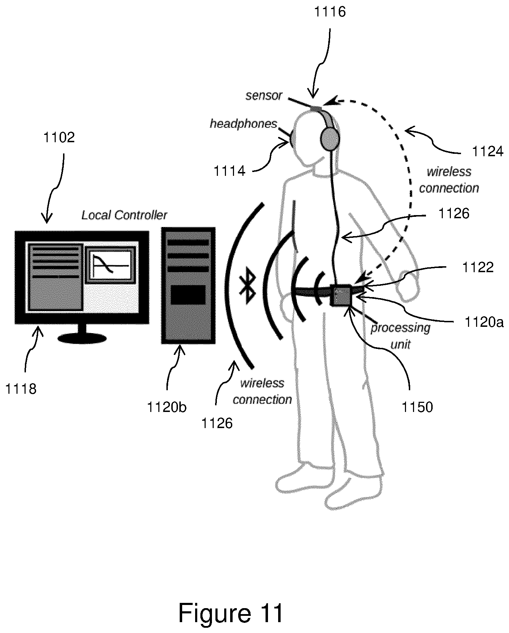

Figures 2(a) and 2(b) show an embodiment of a hearing test system 200, in which the components of the hearing test device 12 of Figure 1, the user input device 17 and display 18 are provided together as a hearing test computer device 202. Figure 11 illustrates a further non-limiting example of a hearing test system, in accordance with an embodiment of the invention. Figure 11 illustrates an embodiment with more than one processing resource and illustrates different communication interfaces between components of the hearing test system.

The hearing test system has a test subject device 1150 that is provided on the test subject. The test subject device may also be referred to as a portable device. The test subject device is configured to be worn by the test subject. In the present embodiment, the test subject device 1150 is provided on a belt 1122 worn around the waist of the test subject. It will be understood that the test subject device 1150 may be provided as a part of a variety of wearable objects. For example, the test subject device may be provided as part of a pendant, a watch, a necklace, a bracelet, sunglasses or other item of clothing. The test subject device may be provided as a portable computing resource that is worn about the body, for example, a smart phone provided in a wearable holder or a smart watch.

The test subject device has a first processing resource 1120a. The hearing test system also has a pair of headphones 1114 and a head-mounted tracking unit 1116 substantially corresponding to headphones 14 and head mounted tracking unit 1116 described with reference to Figure 2. As described in the further detail in the following, the pair of headphones 1114 and head-mounted tracking unit 1116 are connected to the test subject device 1150.

In addition to the test subject device 1150, hearing test system has a hearing test computer 1102 which has a display 1118 and a processor, referred to as the second processing resource 1120b. The display corresponds substantially to display 18 as described with reference to Figure 1 and is intended for use by a hearing test operator. Hearing test computer 1102 also has a user input device (not shown) corresponding to user input device 17 of Figure 1.

Together, the first processing resource 1120a of test subject device 1150 and the second processing resource 1120b of hearing test computer 1120b work together to perform the methods and method steps described with reference to Figures 1 to 10.

The test subject device has a transmitter and receiver for transmitting and receiving data. Likewise, the head-mounted tracking unit 1116 and hearing test computer 1120b have corresponding transmitter/receiver pairs for transmitting and receiving data.

Figure 11 also illustrates a number of communication interfaces between different components of the hearing test system. A first communication interface is provided between the first processing unit 1120a and the headphones 1114. A second communication interface is provided between the first processing unit 1120a and the head-mounted tracking unit 1116. A third communication interface is provided between the first processing unit 1120a of the test subject device 1150 and the second processing unit 1120b of the hearing test computer.

It will be understood that each of the first, second and third communication interfaces may be a wired or a wireless communication interface. In the present embodiment, the first communication interface between the first processing resource 1120a and the headphones 1114 is a wired connection, in this embodiment, the wired connection is a cable. The second communication interface between the first processing resource 1120a and the head-mounted tracking unit 1116 is a wireless connection. The third communication interface between the first processing resource 1120a and the second processing 1120b is a wireless connection.

Any suitable wired/wireless connection may be used. For example, wireless communication may be provided in accordance with the Bluetooth protocol.

In the present embodiment, the first processing resource 1120a is configured to provide audio stimulus signals to the headphones 1114 via the first wired communication interface. Following the movement or absence of movement of the test subject in response to the audio stimulus, the first processing resource 1120a is configured to receive directional response data from the head-mounted tracking unit 1116 via the second, wireless, communication interface. The directional response data is transmitted by the transmitter of the head-mounted tracking unit 1116 and received by the receiver of the test subject device 1150, via the second wireless communication interface.

In the present embodiment, the first processing resource 1120a performs processing required for sound simulation. The processing includes source transformation (laterality exaggeration) using the received directional response data from the head-mounted tracking unit 1116. The first processing resource 1120a therefore processes the directional response data and generates audio stimulus signals based on at least on this processing. The first processing resource 1120a further performs all real-time reactivity to the directional response data in order to simulate an external sound source.

While it will be understood that the first/second/third communication interfaces may be provided as wired or wireless communication interfaces, there are advantages in providing the first, second and/or third communication interfaces as wireless communication interfaces.

In particular, it was found that when using wired connections, cables were producing knocking sounds in the headphones when a test subject moved their head which is undesirable. In addition, the wireless connection promotes and allows increased freedom of movement of the test subject. This combats the psychological tendency of subjects not to move their head due to perception of fragility. This is particularly important as the hearing test relies on patient head movement. Furthermore, during a test, cable entanglement is avoided.

In addition, 360 degree sound delivery may be enabled by using wireless interfaces. By providing 360 degree sound delivery, ambiguity of head movement may be reduced. This allows for easier determination of which sounds have been heard versus which sounds were looked at by chance. The reduced ambiguity of head movement may also lead to increased information yield for a given trial thereby increasing accuracy.

In a further embodiment, a user interface is provided to an operator. The user interface is displayed on a display, for example, display 18 of the hearing test device/system and user input data (or user response data) is received via a user input device, for example, user input device 17. The user interface presents a plurality of questions to the user. The plurality of questions aid in decision making for the hearing test. As a non-limiting list of questions, the plurality of questions include questions regarding ear wax, a question asking about sudden onset of hearing loss within the past 24 hours, the past week, the past month, a question asking about tinnitus (noise, ringing in ears), a question asking the subject age, a question asking about how much the subject engages in social interactions. The questions may include any further questions that help guide decision making on the subject's hearing abilities.

In response to the questions, the user provides answers. The answer data is processed by the processing resource and is used to guide the hearing test. For example, one or more hearing test parameters may be set using the answer data and/or the answer data may be used as part of the hearing test analysis.

In the present embodiment, the questions are asked before the hearing test commences. The answers may be used to manage patient risk, for example, if a patient reports onset hearing loss then they may be directly referred to A&E, as appropriate. The information provided by the answer data may be used to build patient models, thereby to better tailor any result outcomes and streamline care. In some embodiments, a score may be calculated that is representative of a patients risk to inform the clinician.

The above-described embodiments have a number of distinguishing features to known hearing test systems. For example, known systems do not use sound localisation to obtain hearing thresholds or perform machine learning and/or complex statistical analysis on response data, for example, on head movement data, to automate the procedure. Furthermore, known hearing test systems do not perform laterality exaggeration routines. Furthermore, known hearing test systems do not use localisation based paradigms as a method of automated hearing screening.

In the above described embodiments, characteristics and aspects of hearing ability of a test subject are described. Hearing ability will be understood as the ability of a test subject to perceive a sound. Perception in sound may be by detection of vibrations or detection of changes in pressure. Aspects considered in the above-described embodiments include, without limitation, hearing thresholds corresponding to the softest decibel level or volume of sound at a particular frequency that a person reliably responds to. Aspects also include audiograms, and hearing ranges. In the above described embodiments, hearing thresholds for a particular frequency are determined by processing the obtained directional response data in response to an audio stimulus provided at that frequency.

Accordingly, the above description of the specific embodiment is made by way of example only and not for the purposes of limitations. The scope of the invention is defined by the set of appended claims.

**Claims**

1. A device (10) for performing a hearing test on a test subject comprising:
   a processing resource (20) configured to:

   provide audio stimulus signals for an audio output device (14) thereby to provide an audio stimulus to the test subject, wherein the audio stimulus signals are processed such that the provided audio stimulus has a simulated source location;
   obtain directional response data representative of a response of the test subject to the audio stimulus, wherein the directional response data comprises head movement data representative of head movement of the test subject in response to the audio stimulus;
   apply a source location transformation process, wherein the source location transformation process comprises providing a further audio stimulus at a transformed simulated source location being a shifted and/or rotated source location, wherein the transformed simulated source location is based at least on the received directional response data;
   process the obtained directional response data as part of determining the hearing ability of the test subject, wherein the processing of the directional response data comprises detecting one or more pre-determined response features wherein the absence and/or presence of one or more pre-determined response features is representative of or at least indicative of the test subject having heard or having not heard the audio stimulus.

2. A device as claimed in any preceding claim, wherein the processor is configured to select one or more simulated source locations for one or more audio stimuli thereby to encode information in the obtained directional response data, wherein processing said directional response data comprises extracting said encoded information.

3. A device as claimed in claim 1 wherein at least one of a) and b):

   a) the processor is further configured to receive the directional response data in real time and continually apply the source location transformation in response to receiving the directional response data in real time;
   b) the source location transformation process is applied in response to the received directional response data being representative of at least one of: a movement of the test subject and/or a change in a response direction of the test subject.

4. A device as claimed in any preceding claim, wherein the simulated source location is at a selected stimulus direction from the test subject and wherein the processor is configured to receive directional response data and process the received directional response data to determine a response direction, and wherein the source location transformation is based on an angular distance between the determined response direction and the selected stimulus direction, optionally wherein the source location transformation is such that the further audio stimulus is provided at a transformed angular distance from the response direction such that the transformed angular distance is greater than the angular distance between the determined response direction and the selected stimulus direction.

5. A device as claimed in any preceding claim, wherein at least one of a), b) and c):

    a) the source location transformation process is **characterised by** one or more transformation parameters, wherein said transformation parameters are selected such that providing the audio stimulus and/or the further audio stimulus at the transformed simulated source location increases, decreases, maintains and/or induces movement of the head of the test subject, optionally wherein the one or more of the transformation parameters are varied and/or determined during the hearing test based at least on the processing of the directional response data;
    b) the source transformation process is applied in real-time so that the obtained directional response data is representative of an oscillating head movement of the test subject;
    c) the processing resource (20) is further configured to modify the simulated source location of the audio stimulus and/or one or more properties of the audio stimulus based on one or more pre-determined characteristics of the hearing ability of the test subject.

6. A device as claimed in any preceding claim, wherein at least one of a), b), c) and d):

    a) the directional response data represents movement of the test subject that is substantially planar;
    b) the directional response data is parameterised using a single parameter;
    c) the directional response data is representative of an angular distance between a direction faced by the test subject and a direction to the simulated source location;

d) the directional response data comprises time-series data.

7. A device as claimed in any preceding claims wherein the processor is further configured to process the directional response data and/or data derived from the directional response data to determine one or more mathematical and/or statistical properties of the directional response data and to perform an assessment of the likelihood of the stimulus having been heard or not heard based on the determined mathematical and/or statistical properties, optionally wherein the mathematical and/or statistical properties comprise one or more of a), b) and c):

    a) a stationary point in the directional response data;
    b) a shape and/or a rate of change and/or a first, second or higher order derivate of the directional response data;
    c) a first and/or higher order statistical moment in the directional response data.

8. A device as claimed in any preceding claim, wherein the one or more response features comprises at least one of the following:

    a) a feature representative of movement towards and/or away from the stimulus direction;
    b) a feature representative of an absence and/or delay of movement;
    c) a feature representative of oscillation about a fixed position;
    d) a feature representative of a response in a pre-determined time window following the audio stimulus;
    e) a feature representative of initial head movements towards the one or more of the audio stimuli and/or the absence of initial head movements away from one or more of the audio stimuli;
    f) a feature representative of a time elapsed between an audio stimulus and an initial movement of the test subject;
    g) a feature representative of a response direction being within an angular range about the simulated source location;
    h) a feature representative of a reversal of a direction of movement.

9. A device as claimed in any preceding claim, wherein at least one of a), b), c) ,d), e), f):

    a) the audio stimulus signals comprise binaural audio stimulus signals such that the provided audio stimulus comprises binaural audio;
    b) the audio stimulus signals comprise two audio stimulus signals that comprise an interaural level difference and an interaural time difference between the two signals such that the audio output device (14) produces an audio stimulus that is perceived by a test subject with normal hearing ability as if is it coming from the simulated source location;

c) the audio stimulus signals are processed so that the audio stimulus has a frequency in the range 125 Hz to 8kHz;

d) the audio stimulus signals are process so that the audio stimulus has a volume in the range -10 dB SPL to 90 dB SPL;

e) said audio stimulus comprises a sound at a pre-determined frequency and said processing of said obtained directional response data comprises determining a hearing threshold of the test subject at that pre-determined frequency;

f) the processor is further configured to provide a plurality of audio stimuli to the test subject and process the directional response data obtained in response to providing the plurality of audio stimuli thereby to determine at least one aspect of the hearing ability of the test subject.

10. A device as claimed in any preceding claim, further comprising a user interface, wherein the processing resource (20) is configured to provide a plurality of queries to a test subject via the user interface and receive user response data via the user interface wherein the hearing test and/or analysis of the directional response data is based at least on the user response data, optionally wherein the queries comprise at least one of the following:

    a. a query relating to presence or absence of ear wax;

    b. a query relating to hearing difficulties;

    c. a query relating to sudden onset of hearing loss within a time period, for example, the past 24 hours, the past week, the past month;

    c. a query relating to symptoms typical of tinnitus including noise, ringing in ears;

    d. a query relating to test subject age;

    e. a query regarding the level of social interaction by the test subject.

11. A system comprising the device of any of claims 1 to 10 further comprising the audio output device (14), wherein the audio output device (14) is configured to receive said audio stimulus signals and produce said audio stimulus.

12. The system of claim 11, wherein the system further comprises at least one of a), b) and c):

    a) a first communication interface between the audio output device (14) and the processing resource (20) for communicating the audio stimulus signals to the audio output device (14);

    b) a sensor configured to sense a response of the test subject and produce directional response data in response to sensing the response of the test subject during and/or in response to producing said audio stimulus;

    c) a wireless communication interface between the sensor and the processing resource (20) configured to wirelessly transmit the directional response data from the sensor to the processing resource (20).

13. A system as claimed in claim 12, wherein the processing resource (20) comprises a first processing resource and a second processing resource and a wireless communication interface between the first and second processing resources, wherein the first processing resource is configured to provide audio stimulus signals to the audio output device (14) and receive directional response data from the sensor, and the first processing resource is further configured to communicate said directional response data to the second processing resource via the wireless communication resource, optionally wherein the first processing resource is further configured to process said directional response data and perform a source location transformation process comprising providing the further audio stimulus at a transformed simulated source location based at least on the processed directional response data, further optionally wherein the first processing resource is provided as part of a portable device, further optionally, wherein the portable device is a wearable device.

14. A method of performing a hearing test on a test subject comprising:

    providing audio stimulus signals for an audio output device (14) thereby to provide an audio stimulus to the test subject, wherein the audio stimulus signals are processed such that the provided audio stimulus has a simulated source location;

    obtaining directional response data representative of a response of the test subject to the audio stimulus, wherein the directional response data comprises head movement data representative of head movement of the test subject in response to the audio stimulus;

    applying a source location transformation process, wherein the source location transformation process comprises providing a further audio stimulus at a transformed simulated source location, the transformed simulated source location comprising a shifted and/or rotated source location, wherein the transformed simulated source location is based at least on the received directional response data;

processing the obtained directional response data as part of determining the hearing ability of the test subject, wherein the processing of the directional response data comprises detecting one or more pre-determined response features wherein the absence and/or presence of one or more pre-determined response features is representative of or at least indicative of the test subject having heard or having not heard the audio stimulus.

15. A non-transitory computer readable medium comprising instructions operable by a processor to perform a method comprising:

providing audio stimulus signals for an audio output device (14) thereby to provide an audio stimulus to the test subject, wherein the audio stimulus signals are processed such that the provided audio stimulus has a simulated source location;

obtaining directional response data representative of a response of the test subject to the audio stimulus, wherein the directional response data comprises head movement data representative of head movement of the test subject in response to the audio stimulus;

applying a source location transformation process, wherein the source location transformation process comprises providing a further audio stimulus at a transformed simulated source location, the transformed simulated source location comprising a shifted and/or rotated source location, wherein the transformed simulated source location is based at least on the received directional response data;

processing the obtained directional response data as part of determining the hearing ability of the test subject, wherein the processing of the directional response data comprises detecting one or more pre-determined response features wherein the absence and/or presence of one or more pre-determined response features is representative of or at least indicative of the test subject having heard or having not heard the audio stimulus.

**Patentansprüche**

1. Vorrichtung (10) zum Durchführen eines Hörtests an einer Testperson, die Folgendes umfasst:
eine Verarbeitungsressource (20), die für Folgendes konfiguriert ist:

Bereitstellen von Audiostimulussignalen für eine Audio-Ausgabevorrichtung (14), um dadurch einen Audiostimulus für die Testperson bereitzustellen, wobei die Audiostimulussignale so verarbeitet werden, dass der bereitgestellte Audiostimulus einen simulierten Quellort aufweist;
Erhalten von Richtungsantwortdaten, die eine Antwort der Testperson auf den Audiostimulus repräsentieren, wobei die Richtungsantwortdaten Kopfbewegungsdaten umfassen, die Kopfbewegungen der Testperson als Antwort auf den Audiostimulus repräsentieren;
Anwenden eines Quellort-Transformationsprozesses, wobei der Quellort-Transformationsprozess das Bereitstellen eines weiteren Audiostimulus an einem transformierten simulierten Quellort umfasst, der ein verschobener und/oder gedrehter Quellort ist, wobei der transformierte simulierte Quellort mindestens auf den empfangenen Richtungsantwortdaten basiert;
Verarbeiten der erhaltenen Richtungsantwortdaten als Teil des Bestimmens der Hörfähigkeit der Testperson, wobei das Verarbeiten der Richtungsantwortdaten das Erfassen eines oder mehrerer vorbestimmter Antwortmerkmale umfasst, wobei das Fehlen und/oder Vorhandensein eines oder mehrerer vorbestimmter Antwortmerkmale repräsentativ dafür ist oder zumindest darauf hinweist, dass die Testperson den Audiostimulus gehört hat oder nicht gehört hat.

2. Vorrichtung nach einem der vorhergehenden Ansprüche, wobei der Prozessor so konfiguriert ist, dass er einen oder mehrere simulierte Quellorte für einen oder mehrere Audiostimuli auswählt, um dadurch Informationen in den erhaltenen Richtungsantwortdaten zu codieren, wobei das Verarbeiten der genannten Richtungsantwortdaten das Extrahieren der genannten codierten Informationen umfasst.

3. Vorrichtung nach Anspruch 1, wobei mindestens eines von a) und b) gilt:

a) der Prozessor ist ferner dafür konfiguriert, die Richtungsantwortdaten in Echtzeit zu empfangen und die Quellortstransformation als Reaktion auf den Empfang der Richtungsantwortdaten in Echtzeit kontinuierlich anzuwenden;
b) der Quellortstransformationsprozess wird als Reaktion darauf angewendet, dass die empfangenen Richtungsantwortdaten mindestens eines der Folgenden aufweisen: eine Bewegung der Testperson und/oder eine Änderung einer Antwortrichtung der Testperson.

4. Vorrichtung nach einem der vorhergehenden Ansprüche, wobei die simulierte Quelle in einer ausgewählten Stimulusrichtung von der Testperson entfernt ist und wobei der Prozessor dafür konfiguriert ist, Richtungsantwortdaten zu empfangen und die empfangenen Richtungsantwortdaten zu verarbeiten, um eine Antwortrichtung zu bestimmen, und wobei die Quellorttransformation basierend auf einem Winkelabstand zwischen der bestimmten Antwortrichtung und der ausgewählten Stimulusrichtung erfolgt, optional wobei die Quellortstransformation derart ist, dass der weitere Audiostimulus in einem transformierten Winkelabstand von der Antwortrichtung bereitgestellt wird, so dass der transformierte Winkelabstand größer ist als der Winkelabstand zwischen der bestimmten Antwortrichtung und der ausgewählten Stimulusrichtung.

5. Vorrichtung nach einem der vorhergehenden Ansprüche, wobei mindestens eines von a), b) und c) gilt:

    a) der Quellorttransformationsprozess ist durch einen oder mehrere Transformationsparameter gekennzeichnet, wobei die genannten Transformationsparameter so ausgewählt sind, dass das Bereitstellen des Audiostimulus und/oder des weiteren Audiostimulus an dem transformierten simulierten Quellort eine Bewegung des Kopfes der Testperson erhöht, verringert, aufrechterhält und/oder induziert, optional wobei der eine oder die mehreren Transformationsparameter während des Hörtests basierend auf mindestens der Verarbeitung der Richtungsantwortdaten variiert und/oder bestimmt werden;
    b) der Quelltransformationsprozess wird in Echtzeit angewendet, so dass die erhaltenen Richtungsantwortdaten repräsentativ für eine oszillierende Kopfbewegung der Testperson sind;
    c) die Verarbeitungsressource (20) ist ferner dafür konfiguriert, den simulierten Quellort des Audiostimulus und/oder eine oder mehrere Eigenschaften des Audiostimulus basierend auf einer oder mehreren vorbestimmten Eigenschaften der Hörfähigkeit der Testperson zu modifizieren.

6. Vorrichtung nach einem der vorhergehenden Ansprüche, wobei mindestens eines von a), b), c) und d) gilt:

    a) die Richtungsreaktionsdaten stellen eine Bewegung der Testperson dar, die im Wesentlichen planar ist;
    b) die Richtungsreaktionsdaten sind unter Verwendung eines einzigen Parameters parametrisiert;
    c) die Richtungsreaktionsdaten stellen einen Winkelabstand zwischen einer Richtung, in die die Testperson blickt, und einer Richtung zum simulierten Quellort dar;
    d) die Richtungsreaktionsdaten umfassen Zeitreihendaten.

7. Vorrichtung nach einem der vorhergehenden Ansprüche, wobei der Prozessor ferner für Folgendes konfiguriert ist: Verarbeiten der Richtungsreaktionsdaten und/oder aus den Richtungsreaktionsdaten abgeleiteter Daten, um eine oder mehrere mathematische und/oder statistische Eigenschaften der Richtungsreaktionsdaten zu bestimmen, und Durchführen einer Bewertung der Wahrscheinlichkeit, dass der Stimulus gehört oder nicht gehört wurde, basierend auf den bestimmten mathematischen und/oder statistischen Eigenschaften, optional wobei die mathematischen und/oder statistischen Eigenschaften eines oder mehrere der Folgenden umfassen: a), b) und c):

    a) einen stationären Punkt in den Richtungsantwortdaten;
    b) eine Form und/oder eine Änderungsrate und/oder eine Ableitung erster, zweiter oder höherer Ordnung der Richtungsantwortdaten;
    c) ein statistisches Moment erster und/oder höherer Ordnung in den Richtungsantwortdaten.

8. Vorrichtung nach einem der vorhergehenden Ansprüche, wobei das eine oder die mehreren Reaktionsmerkmale mindestens eines der Folgenden umfassen:

    a) ein Merkmal, das eine Bewegung in Richtung auf die Stimulusrichtung und/oder weg von der Stimulusrichtung repräsentiert;
    b) ein Merkmal, das eine Abwesenheit und/oder Verzögerung einer Bewegung repräsentiert;
    c) ein Merkmal, das eine Schwingung um eine feste Position repräsentiert;
    d) ein Merkmal, das eine Reaktion in einem vorbestimmten Zeitfenster nach dem Audiostimulus repräsentiert;
    e) ein Merkmal, das anfängliche Kopfbewegungen in Richtung auf den einen oder die mehreren Audiostimuli und/oder das Fehlen anfänglicher Kopfbewegungen weg von dem einen oder den mehreren Audiostimuli repräsentiert;
    f) ein Merkmal, das eine zwischen einem Audiostimulus und einer anfänglichen Bewegung der Testperson verstrichene Zeit repräsentiert;
    g) ein Merkmal, das eine Reaktionsrichtung innerhalb eines Winkelbereichs um den simulierten Quellort repräsentiert;
    h) ein Merkmal, das eine Umkehrung einer Bewegungsrichtung repräsentiert.

9. Vorrichtung nach einem der vorhergehenden Ansprüche, wobei mindestens eines von a), b), c), d), e), f) gilt:

a) die Audiostimulussignale umfassen binaurale Audiostimulussignale, so dass der bereitgestellte Audiostimulus binauralen Schall umfasst;
b) die Audiostimulussignale umfassen zwei Audiostimulussignale, die eine interaurale Pegeldifferenz und eine interaurale Zeitdifferenz zwischen den beiden Signalen umfassen, so dass die Audio-Ausgabevorrichtung (14) einen Audiostimulus erzeugt, der von einer Testperson mit normalem Hörvermögen so wahrgenommen wird, als käme er von der simulierten Quellort;
c) die Audiostimulussignale werden so verarbeitet, dass der Audiostimulus eine Frequenz im Bereich von 125 Hz bis 8 kHz aufweist;
d) die Audiostimulussignale werden so verarbeitet, dass der Audiostimulus eine Lautstärke im Bereich von -10 dB SPL bis 90 dB SPL aufweist;
e) der genannte Audiostimulus umfasst einen Ton mit einer vorbestimmten Frequenz und die genannte Verarbeitung der genannten erhaltenen Richtungsreaktionsdaten umfasst das Bestimmen einer Hörschwelle der Testperson bei dieser vorbestimmten Frequenz;
f) wobei der Prozessor ferner dafür konfiguriert ist, der Testperson eine Vielzahl von Audiostimuli zuzuführen und die Richtungsreaktionsdaten zu verarbeiten, die als Reaktion auf die Zuführung der Vielzahl von Audiostimuli erhalten werden, um dadurch mindestens einen Aspekt der Hörfähigkeit der Testperson zu bestimmen.

10. Vorrichtung nach einem der vorhergehenden Ansprüche, die ferner eine Benutzerschnittstelle umfasst, wobei die Verarbeitungsressource (20) dafür konfiguriert ist, einer Testperson über die Benutzerschnittstelle eine Vielzahl von Abfragen zu liefern und Benutzerantwortdaten über die Benutzerschnittstelle zu empfangen, wobei der Hörtest und/oder die Analyse der Richtungsantwortdaten mindestens auf den Benutzerantwortdaten basiert, optional wobei die Abfragen mindestens eines der folgenden Elemente umfassen:

a. eine Abfrage bezüglich des Vorhandenseins oder Fehlens von Ohrenschmalz;
b. eine Abfrage bezüglich Hörschwierigkeiten;
c. eine Abfrage bezüglich eines plötzlichen Hörverlusts innerhalb eines Zeitraums, beispielsweise in den letzten 24 Stunden, in der letzten Woche, im letzten Monat;
c. eine Abfrage bezüglich typischer Symptome von Tinnitus, einschließlich Geräusche, Ohrensausen;
d. eine Abfrage bezüglich des Alters der Testperson;

e. eine Abfrage bezüglich des Grades der sozialen Interaktion der Testperson.

11. System, das die Vorrichtung aus einem der Ansprüche 1 bis 10 umfasst und ferner die Audioausgabevorrichtung (14) umfasst, wobei die Audioausgabevorrichtung (14) so konfiguriert ist, dass sie die genannten Audiostimuli empfängt und den genannten Audiostimulus erzeugt.

12. System nach Anspruch 11, wobei das System ferner mindestens eines der folgenden Elemente umfasst: a), b) und c):

a) eine erste Kommunikationsschnittstelle zwischen der Audioausgabevorrichtung (14) und der Verarbeitungsressource (20) zum Kommunizieren der Audiostimulussignale an die Audioausgabevorrichtung (14);
b) einen Sensor, der so konfiguriert ist, dass er eine Reaktion der Testperson erfasst und Richtungsreaktionsdaten als Reaktion auf die Erfassung der Reaktion der Testperson während und/oder als Reaktion auf die Erzeugung des genannten Audiostimuluss erzeugt;
c) eine drahtlose Kommunikationsschnittstelle zwischen dem Sensor und der Verarbeitungsressource (20), die so konfiguriert ist, dass sie die Richtungsreaktionsdaten drahtlos vom Sensor an die Verarbeitungsressource (20) überträgt.

13. System nach Anspruch 12, wobei die Verarbeitungsressource (20) eine erste Verarbeitungsressource und eine zweite Verarbeitungsressource und eine drahtlose Kommunikationsschnittstelle zwischen der ersten und der zweiten Verarbeitungsressource umfasst, wobei die erste Verarbeitungsressource dafür konfiguriert ist, Audiostimulussignale an die Audioausgabevorrichtung (14) zu liefern und Richtungsreaktionsdaten von dem Sensor zu empfangen, und wobei die erste Verarbeitungsressource ferner dafür konfiguriert ist, die genannten Richtungsantwortdaten über die drahtlose Kommunikationsressource an die zweite Verarbeitungsressource zu kommunizieren, optional wobei die erste Verarbeitungsressource ferner dafür konfiguriert ist, die genannten Richtungsantwortdaten zu verarbeiten und einen Quellorttransformationsprozess durchzuführen, der das Bereitstellen des weiteren Audiostimulus an einem transformierten simulierten Quellort umfasst, basierend auf mindestens den verarbeiteten Richtungsantwortdaten, ferner optional wobei die erste Verarbeitungsressource als Teil einer tragbaren Vorrichtung bereitgestellt ist, ferner optional wobei die tragbare Vorrichtung eine am Körper tragbare Vorrichtung ist.

**14.** Verfahren zum Durchführen eines Hörtests an einer Testperson, das Folgendes umfasst:

Bereitstellen von Audiostimulussignalen für eine Audio-Ausgabevorrichtung (14), um dadurch einen Audiostimulus für die Testperson bereitzustellen, wobei die Audiostimulussignale so verarbeitet werden, dass der bereitgestellte Audiostimulus einen simulierten Quellort aufweist; Erhalten von Richtungsantwortdaten, die eine Antwort der Testperson auf den Audiostimulus repräsentieren, wobei die Richtungsantwortdaten Kopfbewegungsdaten umfassen, die Kopfbewegungen der Testperson als Antwort auf den Audiostimulus repräsentieren; Anwenden eines Quellort-Transformationsprozesses, wobei der Quellort-Transformationsprozess das Bereitstellen eines weiteren Audiostimulus an einem transformierten simulierten Quellort umfasst, wobei der transformierte simulierte Quellort einen verschobenen und/oder gedrehten Quellort umfasst, wobei der transformierte simulierte Quellort mindestens auf den empfangenen Richtungsantwortdaten basiert; Verarbeiten der erhaltenen Richtungsantwortdaten als Teil des Bestimmens der Hörfähigkeit der Testperson, wobei das Verarbeiten der Richtungsantwortdaten das Erfassen eines oder mehrerer vorbestimmter Antwortmerkmale umfasst, wobei das Fehlen und/oder Vorhandensein eines oder mehrerer vorbestimmter Antwortmerkmale repräsentativ dafür ist oder zumindest darauf hinweist, dass die Testperson den Audiostimulus gehört hat oder nicht gehört hat.

**15.** Nicht-flüchtiges, computerlesbares Medium, das Anweisungen umfasst, die von einem Prozessor ausgeführt werden können, um ein Verfahren durchzuführen, das Folgendes umfasst:

Bereitstellen von Audiostimulussignalen für eine Audio-Ausgabevorrichtung (14), um dadurch einen Audiostimulus für die Testperson bereitzustellen, wobei die Audiostimulussignale so verarbeitet werden, dass der bereitgestellte Audiostimulus einen simulierten Quellort aufweist; Erhalten von Richtungsantwortdaten, die eine Antwort der Testperson auf den Audiostimulus repräsentieren, wobei die Richtungsantwortdaten Kopfbewegungsdaten umfassen, die Kopfbewegungen der Testperson als Antwort auf den Audiostimulus repräsentieren; Anwenden eines Quellort-Transformationsprozesses, wobei der Quellort-Transformationsprozess das Bereitstellen eines weiteren Audiostimulus an einem transformierten simulierten Quellort umfasst, wobei der transformierte simulierte Quellort einen verschobenen und/oder gedrehten Quellort umfasst, wobei der transformierte simulierte Quellort mindestens auf den empfangenen Richtungsantwortdaten basiert; Verarbeiten der erhaltenen Richtungsantwortdaten als Teil des Bestimmens der Hörfähigkeit der Testperson, wobei das Verarbeiten der Richtungsantwortdaten das Erfassen eines oder mehrerer vorbestimmter Antwortmerkmale umfasst, wobei das Fehlen und/oder Vorhandensein eines oder mehrerer vorbestimmter Antwortmerkmale repräsentativ dafür ist oder zumindest darauf hinweist, dass die Testperson den Audiostimulus gehört hat oder nicht gehört hat.

**Revendications**

**1.** Dispositif (10) destiné à réaliser un test auditif sur un sujet testé, comprenant :
une ressource de traitement (20) configurée pour :

fournir des signaux de stimulus audio à un dispositif de sortie audio (14) afin de fournir ainsi un stimulus audio au sujet testé, dans lequel les signaux de stimulus audio sont traités de sorte que le stimulus audio fourni a un emplacement de source simulé ; obtenir des données de réponse directionnelle représentatives d'une réponse du sujet testé au stimulus audio, dans lequel les données de réponse directionnelle comprennent des données de mouvement de tête représentatives d'un mouvement de tête du sujet testé en réponse au stimulus audio ; appliquer un processus de transformation d'emplacement de source, dans lequel le processus de transformation d'emplacement de source comprend la fourniture d'un stimulus audio supplémentaire au niveau d'un emplacement de source simulé transformé étant un emplacement de source décalé et/ou pivoté, dans lequel l'emplacement de source simulé transformé est basé au moins sur les données de réponse directionnelle reçues ; traiter les données de réponse directionnelle obtenues dans le cadre de la détermination de la capacité auditive du sujet testé, dans lequel le traitement des données de réponse directionnelle comprend la détection d'un ou de plusieurs éléments de réponse prédéterminés, dans lequel l'absence et/ou la présence d'un ou de plusieurs éléments de réponse prédéterminés sont représentatives ou au moins indicatives du fait que le sujet testé a entendu ou n'a pas entendu le stimulus audio.

2. Dispositif selon l'une quelconque des revendications précédentes, dans lequel le processeur est configuré pour sélectionner un ou plusieurs emplacements de source simulés pour un ou plusieurs stimuli audio afin d'encoder ainsi des informations dans les données de réponse directionnelle obtenues, dans lequel le traitement desdites données de réponse directionnelle comprend l'extraction desdites informations encodées.

3. Dispositif selon la revendication 1, dans lequel au moins l'un des points a) et b) :

   a) le processeur est configuré en outre pour recevoir les données de réponse directionnelle en temps réel et appliquer en continu la transformation d'emplacement de source en réponse à la réception des données de réponse directionnelle en temps réel ;
   b) le processus de transformation d'emplacement de source est appliqué en réponse aux données de réponse directionnelle reçues étant représentatives d'au moins un élément parmi : un mouvement du sujet testé et/ou un changement de direction de réponse du sujet testé.

4. Dispositif selon l'une quelconque des revendications précédentes, dans lequel l'emplacement de source simulé est à une direction de stimulus sélectionnée par rapport au sujet testé et dans lequel le processeur est configuré pour recevoir des données de réponse directionnelle et traiter les données de réponse directionnelle reçues pour déterminer une direction de réponse, et dans lequel la transformation d'emplacement de source est basée sur une distance angulaire entre la direction de réponse déterminée et la direction de stimulus sélectionnée, facultativement dans lequel la transformation d'emplacement de source est telle que le stimulus audio supplémentaire est fourni à une distance angulaire transformée de la direction de réponse de sorte que la distance angulaire transformée est supérieure à la distance angulaire entre la direction de réponse déterminée et la direction de stimulus sélectionnée.

5. Dispositif selon l'une quelconque des revendications précédentes, dans lequel au moins l'un des points a), b) et c) :

   a) le processus de transformation d'emplacement de source est **caractérisé par** un ou plusieurs paramètres de transformation, dans lequel lesdits paramètres de transformation sont sélectionnés de sorte que la fourniture du stimulus audio et/ou du stimulus audio supplémentaire au niveau de l'emplacement de source simulé transformé augmente, diminue, maintient et/ou induit le mouvement de la tête du sujet testé, facultativement dans lequel les un ou plusieurs des paramètres de transformation sont modifiés et/ou déterminés pendant le test auditif sur la base au moins du traitement des données de réponse directionnelle ;
b) le processus de transformation de source est appliqué en temps réel de sorte que les données de réponse directionnelle obtenues sont représentatives d'un mouvement de tête oscillant du sujet testé ;
c) la ressource de traitement (20) est configurée en outre pour modifier l'emplacement de source simulé du stimulus audio et/ou une ou plusieurs propriétés du stimulus audio sur la base d'une ou de plusieurs caractéristiques prédéterminées de la capacité auditive du sujet testé.

6. Dispositif selon l'une quelconque des revendications précédentes, dans lequel au moins l'un des points a), b), c) et d) :

   a) les données de réponse directionnelle représentent un mouvement du sujet testé qui est sensiblement planaire ;
   b) les données de réponse directionnelle sont paramétrées à l'aide d'un seul paramètre ;
   c) les données de réponse directionnelle sont représentatives d'une distance angulaire entre une direction à laquelle le sujet testé fait face et une direction vers l'emplacement de source simulé ;
   d) les données de réponse directionnelle comprennent des données de série chronologique.

7. Dispositif selon l'une des revendications précédentes dans lequel le processeur est configuré en outre pour traiter les données de réponse directionnelle et/ou les données dérivées des données de réponse directionnelle afin de déterminer une ou plusieurs propriétés mathématiques et/ou statistiques des données de réponse directionnelle et d'effectuer une évaluation de la probabilité que le stimulus ait été entendu ou non sur la base des propriétés mathématiques et/ou statistiques déterminées, facultativement dans lequel les propriétés mathématiques et/ou statistiques comprennent un ou plusieurs des points : a), b) et c) :

   a) un point stationnaire dans les données de réponse directionnelle ;
   b) une forme et/ou un taux de variation et/ou un dérivé de premier ordre, de deuxième ordre ou d'ordre supérieur des données de réponse directionnelle ;
   c) un moment statistique de premier ordre et/ou d'ordre supérieur dans les données de réponse directionnelle.

8. Dispositif selon l'une quelconque des revendications précédentes, dans lequel les un ou plusieurs éléments de réponse comprennent au moins l'un des suivants :

  a) un élément représentatif d'un mouvement en direction et/ou en provenance de la direction de stimulus ;
  b) un élément représentatif d'une absence et/ou d'un retard de mouvement ;
  c) un élément représentatif de l'oscillation autour d'une position fixe ;
  d) un élément représentatif d'une réponse dans une fenêtre temporelle prédéterminée suivant le stimulus audio ;
  e) un élément représentatif de mouvements de tête initiaux allant vers les un ou plusieurs des stimuli audio et/ou de l'absence de mouvements de tête initiaux s'éloignant d'un ou de plusieurs des stimuli audio ;
  f) un élément représentatif d'un temps écoulé entre un stimulus audio et un mouvement initial du sujet testé ;
  g) un élément représentatif d'une direction de réponse étant dans les limites d'une plage angulaire autour de l'emplacement de source simulé ;
  h) un élément représentatif de l'inversion d'une direction de mouvement.

9. Dispositif selon l'une quelconque des revendications précédentes, dans lequel au moins l'un des points a), b), c), d), e), f) :

  a) les signaux de stimulus audio comprennent des signaux de stimulus audio binauraux de sorte que le stimulus audio fourni comprend un son binaural ;
  b) les signaux de stimulus audio comprennent deux signaux de stimulus audio qui comprennent une différence de niveau interaurale et une différence de temps interaurale entre les deux signaux de sorte que le dispositif de sortie audio (14) produit un stimulus audio qui est perçu par un sujet testé ayant une capacité auditive normale comme s'il provenait de l'emplacement de source simulé ;
  c) les signaux de stimulus audio sont traités de sorte que le stimulus audio a une fréquence dans la plage de 125 Hz à 8 kHz ;
  d) les signaux de stimulus audio sont traités de sorte que le stimulus audio a un volume dans la plage de - 10 dB SPL à 90 dB SPL ;
  e) ledit stimulus audio comprend un son à une fréquence prédéterminée et ledit traitement desdites données de réponse directionnelle obtenues comprend la détermination d'un seuil d'audition du sujet testé à cette fréquence prédéterminée ;
  f) le processeur est configuré en outre pour fournir une pluralité de stimuli audio au sujet testé et traiter les données de réponse directionnelle obtenues en réponse à la fourniture de la pluralité de stimuli audio afin de déterminer au moins un aspect de la capacité auditive du sujet testé.

10. Dispositif selon l'une quelconque des revendications précédentes, comprenant en outre une interface utilisateur, dans lequel la ressource de traitement (20) est configurée pour présenter une pluralité de questions à un sujet testé via l'interface utilisateur et recevoir des données de réponse d'utilisateur via l'interface utilisateur dans lequel le test auditif et/ou l'analyse des données de réponse directionnelle sont basés au moins sur les données de réponse d'utilisateur, facultativement dans lequel les questions comprennent au moins l'une des suivantes :

  a. une question relative à la présence ou à l'absence de cérumen ;
  b. une question relative aux troubles auditifs ;
  c. une question relative à l'apparition soudaine d'une perte auditive au cours d'une période donnée, par exemple, les dernières 24 heures, la semaine dernière ou le mois dernier ;
  c. une question relative à des symptômes typiques des acouphènes, incluant un bruit, un bourdonnement dans les oreilles ;
  d. une question relative à l'âge du sujet testé ;
  e. une question relative au niveau d'interaction sociale du sujet testé.

11. Système comprenant le dispositif selon l'une quelconque des revendications 1 à 10 comprenant en outre le dispositif de sortie audio (14), dans lequel le dispositif de sortie audio (14) est configuré pour recevoir lesdits signaux de stimulus audio et produire ledit stimulus audio.

12. Système selon la revendication 11, dans lequel le système comprend en outre au moins l'un des points a), b) et c) :

  a) une première interface de communication entre le dispositif de sortie audio (14) et la ressource de traitement (20) destinée à communiquer les signaux de stimulus audio au dispositif de sortie audio (14) ;
  b) un capteur configuré pour détecter une réponse du sujet testé et produire des données de réponse directionnelle en réponse à la détection de la réponse du sujet testé pendant et/ou en réponse à la production dudit stimulus audio ;
c) une interface de communication sans fil entre le capteur et la ressource de traitement (20) configurée pour transmettre sans fil les données de réponse directionnelle du capteur à la ressource de traitement (20).

13. Système selon la revendication 12, dans lequel la ressource de traitement (20) comprend une première ressource de traitement et une deuxième ressource de traitement et une interface de communication sans fil entre les première et deuxième ressources de traitement, dans lequel la première ressource de traitement est configurée pour fournir des signaux de stimulus audio au dispositif de sortie audio (14) et recevoir des données de réponse directionnelle du capteur, et la première ressource de traitement est configurée en outre pour communiquer lesdites données de réponse directionnelle à la deuxième ressource de traitement via la ressource de communication sans fil, facultativement dans lequel la première ressource de traitement est configurée en outre pour traiter lesdites données de réponse directionnelle et réaliser un processus de transformation d'emplacement de source comprenant la fourniture du stimulus audio supplémentaire à un emplacement de source simulé transformé sur la base au moins des données de réponse directionnelle traitées, en outre facultativement, dans lequel la première ressource de traitement est fournie comme une partie d'un dispositif portable, en outre facultativement, dans lequel le dispositif portable est un dispositif portable sur soi.

14. Procédé de réalisation d'un test auditif sur un sujet testé comprenant :

la fourniture de signaux de stimulus audio à un dispositif de sortie audio (14) afin de fournir ainsi un stimulus audio au sujet testé, dans lequel les signaux de stimulus audio sont traités de sorte que le stimulus audio fourni a un emplacement de source simulé ;
l'obtention de données de réponse directionnelle représentatives d'une réponse du sujet testé au stimulus audio, dans lequel les données de réponse directionnelle comprennent des données de mouvement de tête représentatives d'un mouvement de tête du sujet testé en réponse au stimulus audio ;
l'application d'un processus de transformation d'emplacement de source, dans lequel le processus de transformation d'emplacement de source comprend la fourniture d'un stimulus audio supplémentaire au niveau d'un emplacement de source simulé transformé, l'emplacement de source simulé transformé comprenant un emplacement de source décalé et/ou pivoté, dans lequel l'emplacement de source simulé transformé est basé au moins sur les données de réponse directionnelle reçues ;
le traitement des données de réponse directionnelle obtenues dans le cadre de la détermination de la capacité auditive du sujet testé, dans lequel le traitement des données de réponse directionnelle comprend la détection d'un ou de plusieurs éléments de réponse prédéterminés dans lequel l'absence et/ou la présence d'un ou de plusieurs éléments de réponse prédéterminés sont représentatives ou au moins indicatives du fait que le sujet testé a entendu ou n'a pas entendu le stimulus audio.

15. Support lisible par ordinateur non transitoire comprenant des instructions pouvant être exécutées par un processeur pour réaliser un procédé comprenant :

la fourniture de signaux de stimulus audio à un dispositif de sortie audio (14) afin de fournir ainsi un stimulus audio au sujet testé, dans lequel les signaux de stimulus audio sont traités de sorte que le stimulus audio fourni a un emplacement de source simulé ;
l'obtention de données de réponse directionnelle représentatives d'une réponse du sujet testé au stimulus audio, dans lequel les données de réponse directionnelle comprend des données de mouvement de tête représentatives d'un mouvement de tête du sujet testé en réponse au stimulus audio ;
l'application d'un processus de transformation d'emplacement de source, dans lequel le processus de transformation d'emplacement de source comprend la fourniture d'un stimulus audio supplémentaire au niveau d'un emplacement de source simulé transformé, l'emplacement de source simulé transformé comprenant un emplacement de source décalé et/ou pivoté, dans lequel l'emplacement de source simulé transformé est basé au moins sur les données de réponse directionnelle reçues ;
le traitement des données de réponse directionnelle obtenues dans le cadre de la détermination de la capacité auditive du sujet testé, dans lequel le traitement des données de réponse directionnelle comprend la détection d'un ou de plusieurs éléments de réponse prédéterminés dans lequel l'absence et/ou la présence d'un ou de plusieurs éléments de réponse prédéterminés sont représentatives ou au moins indicatives du fait que le sujet testé a entendu ou n'a pas entendu le stimulus audio.

Figure 1

Figure 2(a)

Figure 2(b)

Figure 3

Figure 4

Figure 5

Figure 6

Figure 7

Figure 8

Figure 9

Figure 10(a)

Figure 10(b)

Figure 11

**EP 3 957 085 B1**

REFERENCES CITED IN THE DESCRIPTION

**Patent documents cited in the description**

- US 2011019846 A
- US 5825894 A
- US 2016373869 A